# EUROPEAN PATENT APPLICATION

(11) **EP 4 467 140 A1**
(43) Date of publication of application: **27.11.2024**
(21) Application number: 23742945.1
(22) Date of filing: 18.01.2023
(51) Int. Cl.: A61K 31/506, A61K 31/4523, A61K 39/395, A61P 35/00

(54) **PHARMACEUTICAL COMBINATION FOR TREATING TUMORS AND USE THEREOF**

(30) Priority: 21.01.2022 CN 202210071792; 22.09.2022 CN 202211160420
(71) Applicant: Inxmed (Nanjing) Co., Ltd., Nanjing, Jiangsu 210061 (CN)
(72) Inventor: ZHANG, Baoyuan, Nanjing, Jiangsu 210061 (CN); LIU, Xuebin, Nanjing, Jiangsu 210061 (CN); WANG, Zaiqi, Nanjing, Jiangsu 210061 (CN)
(74) Representative: D Young & Co LLP
(86) International application number: PCT/CN2023/072974
(87) International publication number: WO 2023/138630

(57) **Abstract**

An FAK inhibitor, an MEK inhibitor, and an immune checkpoint inhibitor are used in combination to treat tumors, and an FAK inhibitor and an MEK inhibitor are used in combination to treat tumors.

## Description

This application claims the priority of Chinese Patent Application No. 202210071792.6 filed on January 21, 2022 and Chinese Patent Application No. 202211160420.7 filed on September 22, 2022. The disclosures of the above-mentioned Chinese patent applications are hereby incorporated by reference in their entirety as part of this application.

### TECHNICAL FIELD

The present disclosure belongs to the field of medicinal chemistry. Specifically, the present disclosure relates to the treatment of tumors using a focal adhesive kinase (FAK) inhibitor in combination with other drugs.

### BACKGROUND

Tumor is the second largest killer threatening people's health. Immunogenic cell death (ICD) is based on the superposition effect of programmed cell death. An intracellular stress signal may be activated when cancer cells are exposed to chemotherapy or targeted medicaments. This kind of stress signal includes endoplasmic reticulum stress (ER stress) and reactive oxygen species stress (oxidative stress). Under the action of the stress signal, cells will try to repair the stress first. The cells will start the programmed death process, if the damage caused by the stress exceeds the repair ability of the cells. This process is often accompanied by the release of a class of damage associated molecular patterns (DAMPs). This kind of DAMPs will be specifically recognized by pattern recognition receptors on antigen-presenting cells (APC) in the body, which will induce the maturation, differentiation and activation of APC, and then gradually present it to immune cells such as effector T cells, thus making immune cells generate antigen memory. When tumor cells from the same source are found again, immune cells will specifically recognize and kill the tumor cells. The new tumor-specific immune response initiated by ICD can increase the sensitivity to an immune checkpoint inhibitor (ICI), so as to enhance the effect of the immune checkpoint inhibitor, and produce anti-tumor response with lasting immune memory.

FAK, also known as protein tyrosine kinase 2 (PTK2), is a non-receptor tyrosine kinase and a key component of focal adhesion complex. FAK plays an important role in mediating integrin and growth factor signals to regulate the invasion, proliferation and survival of tumor cells.

MEK1, also known as MAP2K1, plays an important role in the MAPK/ERK (RAS-RAF-MEK-ERK) signaling pathway. This signaling pathway can be activated responsively by various stimuli, thereby regulating the proliferation, survival and differentiation of various types of eukaryotic cells, especially in tumor cells where this signaling pathway has been overactivated. Cobimetinib can selectively inhibits MEK1 and MEK2, resulting in decreased ERK1/2 phosphorylation, thereby inhibiting the MAPK/ERK signaling pathway, inhibiting cell proliferation and promoting tumor cell apoptosis. In 2014, the FDA approved the combination of Cobimetinib and vemurafenib (B-RAF kinase inhibitor) for unresectable or metastatic melanoma in patients with B-RAF V600E or V600K mutations. In clinical trials, Cobimetinib subjects experienced adverse reactions such as diarrhea, nausea, vomiting, rash and fever.

It has been observed that some chemotherapy drugs can trigger ICD in cancer cells, and ICD is considered to contribute to the anti-tumor effects of this therapy. Importantly, ICD stimulates the immune system to attack cancer, especially in combination with anti- PD-1 or PD-L1, which can produce a durable anti-tumor effect. Despite some successes in anti-PD-1 combined with chemotherapy and targeted therapy, many clinical trials have failed, especially in ovarian cancer (JAVELIN Ovarian 200) and pembrolizumab combined with lenvatinib in lung cancer. Therefore, there is still a need to further explore drugs that can enhance the induction of ICD by chemotherapy or targeted drugs, improve the response rate of tumor treatment and the possibility of long-term benefit.

### SUMMARY

In one aspect, the present disclosure provides use of an FAK inhibitor, an MEK inhibitor and an immune checkpoint inhibitor in the manufacture of a medicament for treating tumors in a subject, wherein the FAK inhibitor, the MEK inhibitor and the immune checkpoint inhibitor are administered to the subject simultaneously or sequentially.

In another aspect, the present disclosure provides a pharmaceutical combination product of an FAK inhibitor, an MEK inhibitor and an immune checkpoint inhibitor for use in treating tumors in a subject, wherein the FAK inhibitor, the MEK inhibitor and the immune checkpoint inhibitor are administered to the subject simultaneously or sequentially.

In another aspect, the present disclosure provides a method for treating tumors, comprising simultaneously or sequentially administering a therapeutically effective amount of an FAK inhibitor, an MEK inhibitor, and an immune checkpoint inhibitor to a subject in need thereof.

In another aspect, the present disclosure provides a kit or a pharmaceutically acceptable composition comprising: (a) an FAK inhibitor; (b) an MEK inhibitor; and (c) an immune checkpoint inhibitor.

In another aspect, the present disclosure provides use of an FAK inhibitor and an MEK inhibitor in the manufacture of a medicament for treating tumors, wherein the FAK inhibitor is used to enhance the immunogenic cell death induced by the MEK inhibitor.

In another aspect, the present disclosure provides an FAK inhibitor for use in enhancing the immunogenic cell death induced by an MEK inhibitor in treating tumors.

In another aspect, the present disclosure provides a method for treating tumors, comprising administering a therapeutically effective amount of an FAK inhibitor and an MEK inhibitor to a subject in need thereof simultaneously or sequentially, wherein the FAK inhibitor is used to enhance the immunogenic cell death induced by the MEK inhibitor.

In another aspect, the present disclosure provides use of an FAK inhibitor, an MEK inhibitor and an immune checkpoint inhibitor in the manufacture of a combination drug for treating tumors.

In another aspect, the present disclosure provides use of an FAK inhibitor in the manufacture of a combination drug for treating tumors with an MEK inhibitor and an immune checkpoint inhibitor.

In another aspect, the present disclosure provides use of an MEK inhibitor in the manufacture of a combination drug for treating tumors with an FAK inhibitor and an immune checkpoint inhibitor.

In another aspect, the present disclosure provides use of an immune checkpoint inhibitor in the manufacture of a combination drug for treating tumors with an FAK inhibitor and an MEK inhibitor in treating tumors.

In another aspect, the present disclosure provides use of an FAK inhibitor, an MEK inhibitor and an immune checkpoint inhibitor in the manufacture of a medicament for combined treatment of tumors. Another aspect of the present disclosure provides use of an FAK inhibitor in the manufacture of a medicament for combined treatment of tumors with an MEK inhibitor and an immune checkpoint inhibitor.

In another aspect, the present disclosure provides use of an MEK inhibitor in the manufacture of a medicament for treating tumors in combination with an FAK inhibitor and an immune checkpoint inhibitor.

In another aspect, the present disclosure provides use of an immune checkpoint inhibitor in the manufacture of a medicament for treating tumors in combination with an FAK inhibitor and an MEK inhibitor.

In another aspect, the present disclosure provides a kit, which includes: an FAK inhibitor; and an instruction, wherein the instruction indicates that the FAK inhibitor can be used in combination with an MEK inhibitor and an immune checkpoint inhibitor to treat tumors.

In another aspect, the present disclosure provides a kit, which includes: an MEK inhibitor; and an instruction, wherein the instruction indicates that the MEK inhibitor can be used in combination with an FAK inhibitor and an immune checkpoint inhibitor to treat tumors.

In another aspect, the present disclosure provides a kit, which includes: an immune checkpoint inhibitor; and an instruction, wherein the instruction indicates that the immune checkpoint inhibitor can be used in combination with an FAK inhibitor and an MEK inhibitor to treat tumors.

In another aspect, the present disclosure provides a method for treating tumors, comprising administering a therapeutically effective amount of an FAK inhibitor and an MEK inhibitor to a subject in need thereof.

In another aspect, the present disclosure provides a pharmaceutical combination product of an FAK inhibitor and an MEK inhibitor for use in treating tumors in a subject in need thereof.

In another aspect, the present disclosure provides use of an FAK inhibitor and an MEK inhibitor in the manufacture of a combination drug for treating tumors.

In another aspect, the present disclosure provides use of an FAK inhibitor in the manufacture of a combination drug with an MEK inhibitor for use in treating tumors.

In another aspect, the present disclosure provides use of an MEK inhibitor in the manufacture of a combination drug with an FAK inhibitor for use in treating tumors.

In another aspect, the present disclosure provides use of an FAK inhibitor and an MEK inhibitor in the manufacture of a medicament for combined treatment of tumors.

In another aspect, the present disclosure provides use of an FAK inhibitor in the manufacture of a medicament for treating tumors in combination with an MEK inhibitor.

In another aspect, the present disclosure provides use of an MEK inhibitor in the manufacture of a medicament for treating tumors in combination with an FAK inhibitor.

In another aspect, the present disclosure provides a kit, which includes: an FAK inhibitor; and an instruction, wherein the instruction indicates that the FAK inhibitor can be used in combination with an MEK inhibitor to treat tumors.

In another aspect, the present disclosure provides a kit, which includes: an MEK inhibitor; and an instruction, wherein the instruction indicates that the MEK inhibitor can be used in combination with an FAK inhibitor to treat tumors.

Optionally, the FAK inhibitor is IN10018, Defactinib, GSK2256098, PF-00562271, VS-4718, APG-2449, AMP945, AMP886 or pharmaceutically acceptable salts thereof, alternatively the FAK inhibitor is IN10018, Defactinib, AMP945 or pharmaceutically acceptable salts thereof; and further alternatively, the FAK inhibitor is IN10018 or a pharmaceutically acceptable salt thereof, alternatively the FAK inhibitor is IN10018 tartrate, and the structure of IN10018 is as follows: 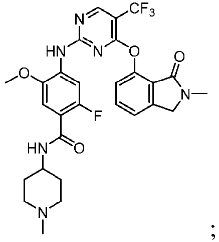 the Defactinib is also known as difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Yasheng Medicine; and the CAS number of AMP945 is 1393653-34-3.

Optionally, the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.

The trametinib is also called Trametinib/Mekinist, with a CAS number of 871700-17-3; the cobimetinib is also called Cobimetinib/Cotellic, with a CAS number of 934660-93-2; the bimetinib is also called Mektovi/binimetinib, with a CAS number of 606143-89-9; the Selumetinib is also called Koselugo/Selumetinib, with a CAS number of 606143-52-6, and the CAS number of its sulfate is 943332-08-9; the PD-325901 is also called Mirdametinib or PD-0325901, with a CAS number of 391210-10-9; the TAK-733 is also called REC-4881, with a CAS number of 1035555-63-5; the HL-085 (HL 085) is an MEK inhibitor developed by Kechow Pharma Inc; E-6201, its CAS number is 603987-35-5; Pimasertib is also called AS703026, its CAS number is 1236699-92-5; Refametinib is also called RDEA119, its CAS number is 923032-37-5; FCN-159 was developed by Fosun Pharma and its subsidiaries.

Optionally, the immune checkpoint inhibitor is an anti-PD-1/PD-L1 antibody, a PD-1/PD-L1 small molecule inhibitor or a TIGIT inhibitor.

Optionally, the immune checkpoint inhibitor is an anti-PD-1/PD-L1 antibody, for example, the anti-PD-1/PD-L1 antibody is pembrolizumab, Tislelizumab, Nivolumab, Toripalimab, Atezolizumab, durvalumab, Avelumab, Camrelizumab, Sintilimab, Cemiplimab, envafolimab, BMS-936559, JS003, SHR-1316, GS-4224, AN-4005, or MX-10181, in particular Atezolizumab.

Optionally, the immune checkpoint inhibitor is a PD-1/PD-L1 small molecule inhibitor, for example, the PD-1/PD-L1 small molecule inhibitor is INCB-086550, Lazertinib, IMMH-010, CA-170, ABSK043 or RRx-001.

Optionally, the immune checkpoint inhibitor is a TIGIT inhibitor, for example, the TIGIT antibody is Ociperfimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036 or IBI-321.

Optionally, the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer. For example, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer or bile duct cancer; further, the tumor is acute myeloid leukemia, melanoma, uveal melanoma, pancreatic cancer, ovarian cancer, colon cancer or colorectal cancer.

### BRIEF DESCRIPTION OF DRAWINGS

In order to more clearly explain the technical solution of examples of the present disclosure, the drawings of examples will be briefly introduced below. Obviously, the drawings in the following description only relate to some examples of the present disclosure, and do not limit the present disclosure.
Fig. 1 shows the 72-hour killing curve and IC50 value of combination with Cobimetinib for melanoma cell A375 after FAK silencing.
Fig. 2 shows the expression levels of proteins related to endoplasmic reticulum stress detected in A375 cells treated with combination with 10 nM and 30 nM Cobimetinib for 6 hours after FAK silencing.
Fig. 3 shows immunofluorescence staining of DAMPs in A375 cells treated with combination with 10 nM Cobimetinib for 48 hours after FAK silencing.
Fig. 4 shows the 72-hour killing curves and IC50 values of IN10018 and Cobimetinib against melanoma cells A375.
Fig. 5 shows the synergistic killing effect of Cobimetinib at different doses combined with 3 µM IN10018 on melanoma A375 cells for 72 hours (* P < 0.05; ** P < 0.01; *** P < 0.001).
Fig. 6 shows the expression levels of proteins related to endoplasmic reticulum stress detected after 1 nM, 3 nM, 10 nM and 30 nM Cobimetinib was co-administered respectively with 3 µM IN10018 for 6 hours.
Fig. 7 shows immunofluorescence staining of DAMPs after 10 nM Cobimetinib and 3 µM IN10018 were co-administered for 48 hours.
Fig. 8 shows the changes in tumor growth of tumor-bearing mice in the BALB/c mouse allograft tumor model of mouse colorectal cancer CT-26 after administration of different test substances.
Fig. 9 shows the animal survival rate of tumor-bearing mice in the BALB/c mouse allograft tumor model of mouse colorectal cancer CT-26 after administration of different test substances.
Fig. 10 shows the changes in the body weight of mice after administration of different doses of drugs and the changes in the body weight of mice after administration of different doses of drugs.
Fig. 11 shows white light microscope pictures of cells taken after the cells were incubated with the drug for 48 hours.
Fig. 12 shows the percentage of CRT-positive CT26 cells after incubation with drugs for 48 hours (Fig. 12a) and the percentage of Annexin V-positive CT26 cells after incubation with drugs for 48 hours (Fig. 12b).
Fig. 13 shows the changes in tumor growth of tumor-bearing mice in the BALB/c mouse allograft tumor model of mouse colorectal cancer CT-26 after administration of different test substances.
Fig. 14 shows the changes in the body weight of mice after administration of different doses of drugs and the changes in the body weight of mice after administration of different doses of drugs.
Fig. 15 shows the percentage of CRT-positive 4T1 cells after incubation with drugs for 48 hours (Fig. 15a) and the percentage of Annexin V-positive 4T1 cells after incubation with drugs for 48 hours (Fig. 15b).
Fig. 16 shows the percentage of CRT-positive CT26 cells after incubation with drugs for 48 hours (Fig. 16a) and the percentage of Annexin V-positive CT26 cells after incubation with drugs for 48 hours (Fig. 16b).

### DETAILED DESCRIPTION

In order to make the purpose, technical solutions and advantages of the examples of the present disclosure clearer, the technical solution of the example of the present disclosure will be described clearly and completely with the attached Figures. Obviously, the described example is a part of examples of the present disclosure, not the whole examples. Based on the described examples of the present disclosure, all other examples obtained by ordinary people in the field without creative labor belong to the scope of protection of the present disclosure.

The present disclosure can be embodied in other specific forms without departing from the basic attributes of the present disclosure. It should be understood that any and all embodiments of the present disclosure can be combined with the technical features of any other embodiment or a plurality of other embodiments to obtain additional embodiments without conflict. The present disclosure includes further embodiments resulting from such combinations.

All publications and patents mentioned in this disclosure are hereby incorporated by reference in their entirety. If the use or terminology used in any publication and patent incorporated by reference conflicts with the use or terminology used in this disclosure, the use and terminology of the present disclosure shall prevail.

The chapter headings used in this disclosure are only for the purpose of organizing articles and should not be interpreted as limitations on the subject.

Unless otherwise specified, all technical and scientific terms used herein have the usual meaning in the field to which the claimed subject matter belongs. If there are multiple definitions of a term, the definition in the present disclosure shall prevail.

Except in working examples or otherwise indicated, all numbers of quantitative properties such as dosage stated in the description and claims should be understood as being modified in all instances by the term "about". It should also be understood that any numerical range recited in this application is intended to include all subranges within the range and any combination of endpoints of the range or subrange.

"Including", "containing", "comprising" or the like used in this disclosure means that the elements appearing before the word cover the elements listed after the word and their equivalents, but do not exclude elements that are not recorded. The term "including", "containing", or "comprising" used herein can be open, semi-closed and closed. In other words, the term also includes "consisting essentially of" or "consisting of".

### Definition

The following terms and symbols used in this application have the following meanings, unless otherwise specified in the context. As used herein, the term "FAK inhibitor" refers to an effective inhibitor of FAK, which can be suitable for mammals, especially humans. In some embodiments, the FAK inhibitor is IN10018, Defactinib, GSK2256098, PF-00562271, VS-4718, APG-2449, AMP945, AMP886 or pharmaceutically acceptable salts thereof, wherein the structure of IN10018 is 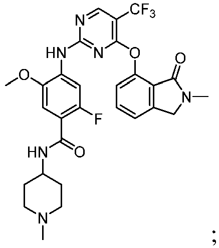 the Defactinib is also known as difatini, and the CAS number is 1345713-71-4; the CAS number of GSK2256098 is 1224887-10-8; the CAS number of PF-00562271 is 717907-75-0; the CAS number of VS-4718 is 1061353-68-1; the APG-2449 is developed by Yasheng Medicine; the CAS number of AMP945 is 1393653-34-3. In some embodiments, alternatively the FAK inhibitor is IN10018, Defactinib, AMP945 or a pharmaceutically acceptable salt thereof, and in some alternative embodiments, the FAK inhibitor is IN10018 or a pharmaceutically acceptable salt thereof, especially IN10018 tartrate.

The term "MEK" as used herein refers to a mitogen-activated protein kinase, a serine-threonine kinase that mediates intracellular signaling involved in the regulation of proteins and cellular functions related to membrane, intracellular and intercellular processes as well as transformation, proliferation/growth, differentiation, survival and death.

As used herein, the term "MEK inhibitor" refers to a compound or drug that reduces or decreases MEK-dependent cell signaling/function, and reduces or decreases tumor cell proliferation/growth associated with MEK. Examples of MEK inhibitors include, but are not limited to, trametinib, cobimetinib, bimetinib, Selumetinib, PD-325901, TAK-733, HL-085, E-6201, Pimasertib, Refametinib, FCN-159, or a pharmaceutically acceptable salt thereof. In some embodiments, the MEK inhibitor is cobimetinib, HL-085, or a pharmaceutically acceptable salt thereof.

The trametinib is also known as Trametinib/Mekinist, with a CAS number of 871700-17-3; the cobimetinib is also known as Cobimetinib /Cotellic, with a CAS number of 934660-93-2; the bimetinib is also known as Mektovi/binimetinib, with a CAS number of 606143-89-9; the Selumetinib is also known as Koselugo/Selumetinib, with a CAS number of 606143-52-6, and the CAS number of its sulfate is 943332-08-9; the PD-325901 has a CAS number of 391210-10-9; the TAK-733 has a CAS number of 1035555-63-5; the HL -085 (HL 085) is an MEK inhibitor developed by Kechow Pharma Inc (Shanghai Kezhou Pharmaceutical Research and Development Co., Ltd.); E-6201, its CAS number is 603987-35-5; Pimasertib, also known as AS703026, its CAS number is 1236699-92-5; Refametinib, also known as RDEA119, its CAS number is 923032-37-5; FCN-159 was developed by Fosun Pharma and its subsidiaries.

As used herein, the term "immune checkpoint inhibitor" refers to a medicament that can improve the immune system activity by regulating immune checkpoint pathways (such as PD-1, TIGIT, CTLA-4, LAG-3, TIM-3, etc.). In some embodiments, the immune checkpoint inhibitor is an antagonist of the PD-1/PD-L1 (programmed cell death protein 1) pathway (also called "PD-1 inhibitor") or a TIGIT inhibitor. PD-1 inhibitors are also referred to as PD-1/PD-L1 inhibitors in this disclosure. For example, in the therapeutic methods, medicaments and uses of the present disclosure, the PD-1/PD-L1 inhibitor is a anti-PD-1/PD-L1 antibody, including, but not limited to, pabolizumab (KERIDA/Keytruda/K medicament), Tislelizumab (Baizean), Nivolumab, Toripalimab (Tuoyi), durvalumab (Imfinzi), Avelumab (Bavencio), Atezolizumab (MPDL 3280A/Tecentriq/T medicament), BMS-936559 (fully humanized IgG4 monoclonal antibody against PD-L1), GS-4224, AN-4005 or MX-10181. In some alternative embodiments, the PD-1 inhibitor is Toripalimab. In some alternative embodiments, the PD-1 inhibitor is Atezolizumab. In some embodiments, PD-1 inhibitors are used to treat human subjects. In some embodiments, PD-1 is human PD-1. PD-1/PD-L1 inhibitors also include PD-1/PD-L1 small molecule inhibitors, such as INCB-086550, Lazertinib, IMMH-010, CA-170, ABSK043 or RRx-001. TIGIT (also known as WUCAM, Vstm3, VSIG9) is a receptor of the Ig superfamily and is a new immune checkpoint besides PD-1/PD-L1. For example, in the therapeutic methods, medicaments and uses of the present disclosure, the immune checkpoint inhibitor is a TIGIT inhibitor (such as a TIGIT antibody), including, but not limited to, Ociperlimab (BGB-A1217), Vibostolimab, domvanalimab (AB154), Tiragolumab, Belrestotug, Etigilimab, ONO-4686, JS-006, AZD-2936, HLX-301, SEA-TGT, M-6223, IBI-939, COM-902, AB-308, AGEN-1777, AK-127, BAT-6021, BAT-6005, ASP-8374, PM-1022, BMS-986207, HB0036, or IBI-321. In some embodiments, the TIGIT inhibitors are used to treat human subjects. In order to avoid ambiguity, all antibodies in the present disclosure include double antibodies.

As used herein, "pharmaceutical combination" or "pharmaceutical combination product" can refer to the case of a fixed combination in the form of a dosage unit (for example, all pharmaceutical active ingredients exist in one dosage form) or the case of a product that is administered in combination in a complete kit, and the case of a combination of a medicament and instructions indicating that the medicament can be used in combination with one or more other medicaments.

As used herein, "combination therapy" or "combined medicament" means that a medicament is used in combination with one or more other medicaments to treat a disease, including both the combination of one medicament and one or more other medicaments and the combination of one medicament and instructions indicating that the medicament can be used in combination with one or more other medicaments.

In this application, "simultaneous or sequential administration" refers to the simultaneous administration of two or more medicaments or sequential administration of two or more medicaments at a certain time interval within an administration cycle (for example, within 4 weeks, 3 weeks, 2 weeks, 1 week or 24 hours). The modes of administration (for example, oral administration, intravenous administration, intramuscular administration or subcutaneous administration) may be the same or different, and the frequency/cycle of administration of two or more medicaments may be the same or different. When the therapeutic method, product or use of the present disclosure involves two medicaments, the two medicaments can be administered simultaneously or separately at a certain interval. When the therapeutic method, product or use of the present disclosure involves three medicaments, the three medicaments can be administered at the same time point, or two medicaments can be administered at one time point and the remaining one medicament can be administered at another time point, or each of the three medicaments can be administered at different time points.

In some embodiments, the PD-1/PD-L1 inhibitor is administered intravenously (e.g., as intravenous infusion), subcutaneously or orally. Alternatively, the PD-1/PD-L1 inhibitor is administered by intravenous infusion.

The ability of immune checkpoint inhibitors to treat cancer depends on the existence of tumor antigen-specific T cells in a tumor tissue. This requires tumor tissues to express antigens that distinguish themselves from their untransformed counterparts, for example, through a new protein product called neoantigen. The neoantigen load of tumor is closely related to immunogenicity and sensitivity (for example, sensitivity to checkpoint inhibitor therapy), which means that tumors with poor immunogenicity should be resistant to these medicaments to a great extent. Therapeutics for releasing tumor antigens that can be ingested by APC, such as those that induce immunogenic cell death (ICD), may promote effective anti-tumor immunity, especially when further combined with a checkpoint inhibitor.

As used herein, the term "treatment" refers to the administration of one or more pharmaceutical substances to a subject suffering from a disease or having symptoms of the disease in order to cure, alleviate, mitigate, change, treat, improve, ameliorate or influence the disease or symptoms of the disease. In some embodiments, the disease is a tumor or cancer.

The term "tumor" as used herein refers to an abnormal lesion formed when the cells of local tissues lose normal regulation of their growth at the genetic level under the influence of various tumorigenic factors, resulting in abnormal proliferation of their clonal types. Examples include, but are not limited to: Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer or bile duct cancer; yet further, the tumor is acute myeloid leukemia, melanoma, uveal melanoma, pancreatic cancer, ovarian cancer, colon cancer or colorectal cancer.

As used herein, the term "subject" or "patient" refers to mammals and non-mammals. Mammals refer to any member of mammals, including but not limited to: human; non-human primates, such as chimpanzees and other apes and monkey species; farm animals such as cattle, horses, sheep, goats and pigs; domestic animals such as rabbits, dogs and cats; laboratory animals, including rodents, such as rats, mice and guinea pigs, etc. Examples of non-mammals include but are not limited to birds, etc. The term "subject" is not limited to a specific age or gender. In some embodiments, the subject is a human.

As used herein, the term "pharmaceutically acceptable" means non-toxic, biologically tolerable and suitable for administration to a subject.

As used herein, the term "pharmaceutically acceptable salt" refers to non-toxic and biologically tolerable acid addition salts suitable for administration to subjects, including but not limited to: acid addition salts formed with inorganic acids, such as hydrochloride, hydrobromide, carbonate, bicarbonate, phosphate, sulfate, sulfite, nitrate, etc.; and acid addition salts with organic acids, such as formate, acetate, malate, maleate, fumarate, tartrate, succinate, citrate, lactate, methanesulfonate, p-toluenesulfonate, 2-hydroxyethyl sulfonate, benzoate, salicylate, stearate and salts formed with alkanedicarboxylic acids of formula HOOC-(CH₂)ₙ-COOH (where n is 0-4), etc.

Furthermore, a pharmaceutically acceptable acid addition salt can be obtained by dissolving a free base in a suitable solvent and treating the solution with an acid according to a conventional operation for preparing an acid addition salt from a basic compound. Those skilled in the art can determine various synthetic methods that can be used to prepare nontoxic pharmaceutically acceptable acid addition salts without redundant experiments.

As used herein, the term "pharmaceutically acceptable composition" means that it must be chemically and/or toxicologically compatible with other ingredients included in the manufacture, and/or compatible with the subject to be treated. As used herein, the term "therapeutically effective amount" refers to an amount generally sufficient to produce a beneficial therapeutic effect on a subject. The therapeutically effective amount of the present disclosure can be determined by conventional methods (such as modeling, dose escalation study or clinical trial) combined with conventional influencing factors (such as administration mode, pharmacokinetics of compounds, severity and course of diseases, medical history of subjects, health status of subjects, response degree of subjects to medicaments, etc.).

As used herein, the term "inhibition" refers to a decrease in the baseline activity of a biological activity or process.

As used herein, the term "kit" refers to a box for containing chemical reagents for detecting chemical components, medicament residues, virus types and the like. The kit of the present disclosure can include (i) one, two or three of an FAK inhibitor, an MEK inhibitor and an immune checkpoint inhibitor; and (ii) an instruction indicating that the FAK inhibitor, the MEK inhibitor and the immune checkpoint inhibitor can be used to treat tumors in the subject. In one embodiment, the kit comprises (i) an FAK inhibitor; and (ii) an instruction indicating that the FAK inhibitor, the MEK inhibitor and the immune checkpoint inhibitor can be used to treat tumors in a subject. In one embodiment, the kit includes (i) an MEK inhibitor; and (ii) an instruction indicating that the FAK inhibitor, the MEK inhibitor and the immune checkpoint inhibitor can be used to treat tumors in a subject. In one embodiment, the kit comprises (i) an immune checkpoint inhibitor; and (ii) an instruction indicating that the FAK inhibitor, the MEK inhibitor and the immune checkpoint inhibitor can be used to treat tumors in a subject. In one embodiment, the kit comprises (i) an FAK inhibitor, an MEK inhibitor and an immune checkpoint inhibitor; and (ii) an instruction indicating that the FAK inhibitor, the MEK inhibitor and the immune checkpoint inhibitor can be used to treat tumors in a subject. In one embodiment, the kit comprises (i) an FAK inhibitor; and (ii) an instruction indicating that the FAK inhibitor and the MEK inhibitor can be used to treat tumors in a subject. In one embodiment, the kit includes (i) an MEK inhibitor; and (ii) an instruction indicating that the MEK inhibitor and the FAK inhibitor can be used to treat tumors in a subject.

The compounds of a kit can be contained in separate containers. Optionally, two or more compounds are contained in the same container. For example, the kit may include a first container, a second container, a third container, and a package insert, wherein the first container includes at least one dose of a medicament including an FAK inhibitor, the second container includes at least one dose of an MEK inhibitor, and the third container includes at least one dose of a medicament including an immune checkpoint inhibitor, and the package insert includes instructions of treating a tumor in a subject with the medicament. The first container, the second container and the third container may contain the same or different shapes (e.g., vials, syringes and bottles) and/or materials (e.g., plastic or glass). The kit can also include other materials that can help to administer medicaments, such as diluents, filters, IV bags and lines, needles and syringes.

The exact amount of the FAK inhibitor, the MEK inhibitor and the immune checkpoint inhibitor to be administered to a subject will depend on various factors, such as a given medicament or compound, pharmaceutical preparation, route of administration, type of disease, symptoms, identity of the subject or host to be treated, etc., but it can still be routinely determined by those skilled in the art. For example, determining the effective amount also depends on the degree, severity and type of cell proliferation. The skilled person will be able to determine the appropriate dosage based on these and other factors.

The FAK inhibitor, the MEK inhibitor and the immune checkpoint inhibitor can be administered in an appropriate manner, such as oral administration, intravenous administration, intramuscular administration, or subcutaneous administration.

For example, when administered orally, the medicament can be administered orally together with a pharmaceutically acceptable carrier such as an inert diluent or an absorbable edible carrier. They can be encapsulated in hard-shell or soft-shell gelatin capsules, can be pressed into tablets, or can be directly mixed with patients' food. For example, medicaments can be combined with one or more excipients and used in the form of ingestible tablets, oral tablets, lozenges, capsules, elixirs, suspensions, syrups or wafers. Tablets, lozenges, pills, capsules, and the like may further include a binder, such as tragacanth gum, arabic gum, corn starch or gelatin; an excipient, such as dicalcium phosphate; a disintegrator, such as corn starch, potato starch, alginic acid, and the like; a lubricant, such as magnesium stearate; or a sweetener, such as sucrose, fructose, lactose or aspartame; or a flavoring agent.

For example, when administered intravenously or intraperitoneally by infusion or injection, the medicament solution can be prepared in water, optionally mixed with a non-toxic surfactant.

Exemplary pharmaceutical dosage forms for injection or infusion include a sterile aqueous solution, a dispersion, or a sterile powder containing an active ingredient, which is suitable for temporarily preparing a sterile injection or infusion solution or dispersion. In any case, the final dosage form should be sterile, fluid and stable under the conditions of production and storage.

Sterile injection solution can be prepared by mixing the required amount of medicaments with various other components mentioned above into a suitable solvent, and then filtering and sterilizing the mixture. For sterile powder used to prepare sterile injection solution, the alternative preparation methods can be vacuum drying and freeze drying techniques, which can produce powder of active ingredient plus any other required ingredients existing after previous sterile filtration.

The amount of the FAK inhibitor, the MEK inhibitor and the immune checkpoint inhibitor required for treatment can vary not only with the specific reagents selected, but also with the route of administration, the nature of the diseases to be treated, and the age and condition of the patients, and can be finally decided by the attending physician or clinician. However, in general, the dosage can be in the range of about 0.1 to about 50mg/kg body weight per day.

The FAK inhibitor is administered in a dosage range of 5 mg/day to 300 mg/day in adults. In a specific embodiment, IN10018 or a pharmaceutically acceptable salt thereof is administered in a dosage range of 5 mg/day to 100 mg/day in adults, and the dosage is calculated as the free base.

The MEK inhibitor is administered in a dosage range of 2-250 mg per day in adults. In a specific embodiment, cobimetinib or a pharmaceutically acceptable salt thereof is administered in a dosage range of 2-250 mg per day in adults, and the dosage is calculated as cobimetinib; HL-0 85 or a pharmaceutically acceptable salt thereof is administered in a dosage range of 2-250 mg per day in adults, and the dosage is calculated as HL-0 85.

The immune checkpoint inhibitor is administered each time at a dose of 2-10mg/kg or 50-1200mg for adults, and once every 2 to 3 weeks. In a specific embodiment, the immune checkpoint inhibitor is administered each time at a dose of 3-10mg/kg or 100-1200mg for adults and once every two to three weeks.

Technical and scientific terms not specifically defined used herein have the meanings commonly understood by those skilled in the art to which this disclosure belongs.

In some embodiments, the present disclosure also discloses:
1. An FAK inhibitor, an MEK inhibitor and an immune checkpoint inhibitor, which are used in a method for treating tumors in a subject, wherein the FAK inhibitor, the MEK inhibitor and the immune checkpoint inhibitor are administered to the subject simultaneously or sequentially.
2. The compound for use according to embodiment 1, wherein the FAK inhibitor is IN10018, Defactinib, GSK2256098, PF-00562271, VS-4718, APG-2449, AMP945, AMP886 or a pharmaceutically acceptable salt thereof, alternatively the FAK inhibitor is IN10018, Defactinib, AMP945 or a pharmaceutically acceptable salt thereof, further alternatively the FAK inhibitor is IN10018 or a pharmaceutically acceptable salt thereof, alternatively the FAK inhibitor is IN10018 tartrate, and the structure of IN10018 is: 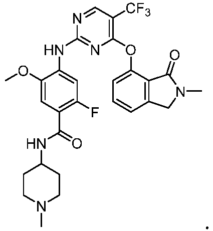
3. The compound for use according to embodiment 1 or 2, wherein the MEK inhibitor is trametinib, cobimetinib, bimetinib, Selumetinib, PD-325901, TAK-733, HL-085 or a pharmaceutically acceptable salt thereof, alternatively the MEK inhibitor is cobimetinib, HL-085 or a pharmaceutically acceptable salt thereof.
4. The compound for use according to any one of embodiments 1-3, wherein the immune checkpoint inhibitor is an anti-PD-1/PD-L1 antibody, a PD-1/PD-L1 small molecule inhibitor or a TIGIT inhibitor.
5. The compound for use according to any one of embodiments 1 to 4, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.
6. The compound for use according to any one of embodiments 1 to 5, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
7. A kit or a pharmaceutically acceptable composition, comprising:
   (a) an FAK inhibitor;
   (b) an MEK inhibitor; and
   (c) an immune checkpoint inhibitor.
8. The kit or composition according to embodiment 7, wherein the FAK inhibitor is IN10018, Defactinib, GSK2256098, PF-00562271, VS-4718, APG-2449, AMP945, AMP886 or a pharmaceutically acceptable salt thereof, alternatively the FAK inhibitor is IN10018, Defactinib, AMP945 or a pharmaceutically acceptable salt thereof, further alternatively the FAK inhibitor is IN10018 or a pharmaceutically acceptable salt thereof, especially IN10018 tartrate, wherein the IN10018 has a structure of: 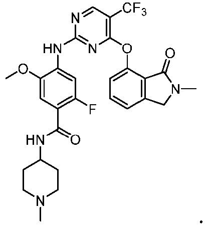
9. The kit or composition according to embodiment 7 or 8, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
10. The kit or composition according to any one of embodiments 7 to 9, wherein the immune checkpoint inhibitor is an anti-PD-1/PD-L1 antibody, a PD-1/PD-L1 small molecule inhibitor, or a TIGIT inhibitor.
11. The kit or composition according to any one of embodiments 7-10, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.
12. The kit or composition according to any one of embodiments 7 to 11 for use as a medicament.
13. The kit or composition according to any one of embodiments 7 to 12, for use in treating tumors.
14. The kit or composition according to embodiment 13, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
15. A method of treating tumors in a subject, wherein the method comprises administering a compound of the kit or composition according to any one of embodiments 7-12 to the subject simultaneously or sequentially.
16. The method of embodiment 15, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
17. An FAK inhibitor for use in enhancing immunogenic cell death induced by the MEK inhibitor.
18. The FAK inhibitor for use according to embodiment 17, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
19. The FAK inhibitor for use according to embodiment 17 or 18, wherein the FAK inhibitor is IN10018, Defactinib, GSK2256098, PF-00562271, VS-4718, APG-2449, AMP945, AMP886 or a pharmaceutically acceptable salt thereof, alternatively, the FAK inhibitor is IN10018, Defactinib, AMP945 or a pharmaceutically acceptable salt thereof, and further alternatively the FAK inhibitor is IN10018, or a pharmaceutically acceptable salt thereof, especially IN10018 tartrate, wherein the IN10018 has a structure of: 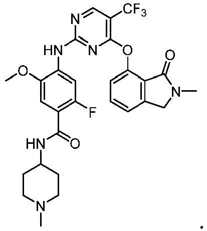
20. The FAK inhibitor for use according to any one of embodiments 17 to 19, wherein the FAK inhibitor and the MEK inhibitor are administered simultaneously or sequentially.
21. A method for treating tumors in a subject, wherein the method comprises administering to the subject a therapeutically effective amount of an FAK inhibitor, an MEK inhibitor, and an immune checkpoint inhibitor simultaneously or sequentially.
22. The method according to embodiment 21, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945 or a pharmaceutically acceptable salt thereof, further alternatively, the FAK inhibitor is IN10018, or a pharmaceutically acceptable salt thereof, alternatively, the FAK inhibitor is IN 100 18 tartrate, wherein the IN 100 18 has a structure of: 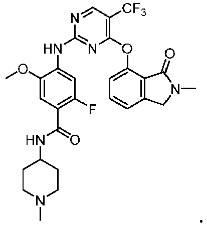
23. The method according to embodiment 21 or 22, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
24. The method according to any one of embodiments 21-23, wherein the immune checkpoint inhibitor is an anti-PD-1/PD-L1 antibody, a PD-1/PD-L1 small molecule inhibitor, or a TIGIT inhibitor.
25. The method according to any one of embodiments 21-24, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.
26. The method according to any one of embodiments 21 to 25, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
27. An FAK inhibitor, an MEK inhibitor and an immune checkpoint inhibitor for use in a method for treating tumors by increasing immunogenic cell death in a subject, wherein the FAK inhibitor, the MEK inhibitor and the immune checkpoint inhibitor are administered to the subject simultaneously or sequentially.
28. The compound for use according to embodiment 27, wherein the FAK inhibitor is IN10018, defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and further alternatively, the FAK inhibitor is IN10018, or a pharmaceutically acceptable salt thereof, alternatively, the FAK inhibitor is IN10018 tartrate, wherein the IN10018 has a structure of: 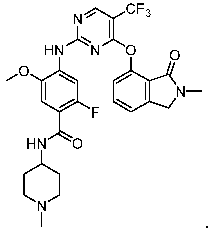
29. The method according to embodiment 27 or 28, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
30. The compound for use according to any one of embodiments 27-29, wherein the immune checkpoint inhibitor is an anti-PD-1/PD-L1 antibody, a PD-1/PD-L1 small molecule inhibitor or a TIGIT inhibitor.
31. The compound for use according to any one of embodiments 27 to 30, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.
32. The compound for use according to any one of embodiments 27 to 31, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
33. A method for treating tumors by increasing immunogenic cell death in a subject, wherein the method comprises administering a therapeutically effective amount of an FAK inhibitor, an MEK inhibitor, and an immune checkpoint inhibitor to the subject simultaneously or sequentially.
34. The method according to embodiment 33, wherein the FAK inhibitor is IN10018, Defactinib, GSK2256098, PF-00562271, VS-4718, APG-2449, AMP945, AMP886, or a pharmaceutically acceptable salt thereof, alternatively, the FAK inhibitor is IN10018, Defactinib, AMP945, or a pharmaceutically acceptable salt thereof, and further alternatively, the FAK inhibitor is IN10018, or a pharmaceutically acceptable salt thereof, alternatively, the FAK inhibitor is IN10018 tartrate, wherein the IN10018 has a structure of: 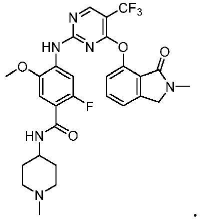
35. The method according to embodiment 33 or 34, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
36. The method according to any one of embodiments 33-35, wherein the immune checkpoint inhibitor is an anti-PD-1/PD-L1 antibody, a PD-1/PD-L1 small molecule inhibitor, or a TIGIT inhibitor.
37. The method according to any one of embodiments 33 to 36, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.
38. The method according to any one of embodiments 33 to 37, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
39. Use of an FAK inhibitor in the manufacture of a medicament for treating tumors in a subject, wherein the FAK inhibitor, MEK inhibitor and immune checkpoint inhibitor are administered to the subject simultaneously or sequentially.
40. The use according to embodiment 39, wherein the FAK inhibitor is IN10018, Defactinib, GSK2256098, PF-00562271, VS-4718, APG-2449, AMP945, AMP886 or a pharmaceutically acceptable salt thereof, alternatively the FAK inhibitor is IN10018, Defactinib, AMP945 or a pharmaceutically acceptable salt thereof, further alternatively the FAK inhibitor is IN10018 or a pharmaceutically acceptable salt thereof, especially IN10018 tartrate, wherein the IN10018 has a structure of: 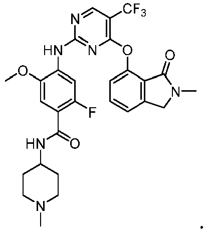
41. The use according to embodiment 39 or 40, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
42. The use according to any one of embodiments 39-41, wherein the immune checkpoint inhibitor is an anti-PD-1/PD-L1 antibody, a PD-1/PD-L1 small molecule inhibitor or a TIGIT inhibitor.
43. The use according to any one of embodiments 39 to 42, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1 /PD-L1 antibody or a PD-1/PD-L1 small molecule inhibitor, especially atezolizumab.
44. The use according to any one of embodiments 39-43, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
45. Use of an MEK inhibitor in the manufacture of a medicament for treating tumors in a subject, wherein an FAK inhibitor, the MEK inhibitor and an immune checkpoint inhibitor are administered to the subject simultaneously or sequentially.
46. The use according to embodiment 45, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
47. The use according to embodiment 45 or 46, wherein the FAK inhibitor is IN10018, Defactinib, GSK2256098, PF-00562271, VS-4718, APG-2449, AMP945, AMP886, or a pharmaceutically acceptable salt thereof, alternatively the FAK inhibitor is IN10018, Defactinib, AMP945, or a pharmaceutically acceptable salt thereof, and further alternatively the FAK inhibitor is IN10018, or a pharmaceutically acceptable salt thereof, especially IN10018 tartrate, wherein the IN10018 has a structure of: 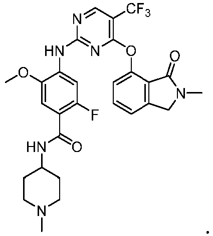
48. The use according to any one of embodiments 45-47, wherein the immune checkpoint inhibitor is an anti-PD-1/PD-L1 antibody, a PD-1/PD-L1 small molecule inhibitor or a TIGIT inhibitor.
49. The use according to any one of embodiments 45 to 48, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.
50. The use according to any one of embodiments 45 to 49, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.
51. Use of an immune checkpoint inhibitor in the manufacture of a medicament for treating tumors in a subject, wherein an FAK inhibitor, an MEK inhibitor and the immune checkpoint inhibitor are administered to the subject simultaneously or sequentially.
52. Use of an FAK inhibitor, an MEK inhibitor and an immune checkpoint inhibitor in the manufacture of a combination drug for treating tumors.
53. Use of an FAK inhibitor in the manufacture of a combination drug for treating tumors with an MEK inhibitor and an immune checkpoint inhibitor.
54. Use of an MEK inhibitor in the manufacture of a combination drug for treating tumors with an FAK inhibitor and an immune checkpoint inhibitor.
55. Use of an immune checkpoint inhibitor in the manufacture of a combination drug for treating tumors with an FAK inhibitor and an MEK inhibitor.
56. Use of an FAK inhibitor, an MEK inhibitor and an immune checkpoint inhibitor in the manufacture of a medicament for combined treatment of tumors.
57. Use of an FAK inhibitor in the manufacture of a medicament for treating tumors in combination with an MEK inhibitor and an immune checkpoint inhibitor.
58. Use of an MEK inhibitor in the manufacture of a medicament for treating tumors in combination with an FAK inhibitor and an immune checkpoint inhibitor.
59. Use of an immune checkpoint inhibitor in the manufacture of a medicament for treating tumors in combination with an FAK inhibitor and an MEK inhibitor.
60. The use according to any one of embodiments 51 to 59, wherein the FAK inhibitor is IN10018, Defactinib, GSK2256098, PF-00562271, VS-4718, APG-2449, AMP945, AMP886, or a pharmaceutically acceptable salt thereof, alternatively the FAK inhibitor is IN10018, Defactinib, AMP945 or a pharmaceutically acceptable salt thereof, and further alternatively the FAK inhibitor is IN10018 or a pharmaceutically acceptable salt thereof, especially IN10018 tartrate, wherein the IN10018 has a structure of: 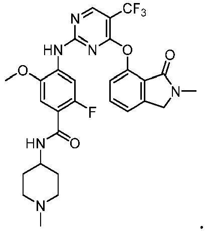
61. The use according to any one of embodiments 51 to 60, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.
62. The use according to any one of embodiments 51-61, wherein the immune checkpoint inhibitor is an anti-PD-1/PD-L1 antibody, a PD-1/PD-L1 small molecule inhibitor or a TIGIT inhibitor.
63. The use according to any one of embodiments 51-62, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.
64. The use according to any one of embodiments 51 to 63, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colorectal cancer, esophageal cancer, hepatocellular carcinoma, ovarian cancer, fibrosarcoma, gastric cancer, non-small cell lung cancer, or bile duct cancer; yet further, the tumor is acute myeloid leukemia, melanoma, uveal melanoma, pancreatic cancer, ovarian cancer, colon cancer or colorectal cancer.

### Examples

The following examples are provided to further illustrate the present disclosure. It should be understood that these examples are only used to illustrate the present disclosure and are not used to limit the scope of the present disclosure.

The assay methods without specific conditions in the following examples can be carried out according to the conventional conditions of this kind of reaction or according to the conditions suggested by the manufacturer.

The assay materials and reagents used in the following examples are all commercially available unless otherwise specified. The abbreviations used in the examples have the following meanings:

| Abbreviation | Name |
|---|---|
| siRNA | Small interfering RNA |
| DMSO | Dimethyl sulfoxide |
| COMBO | Combination |
| HMGB1 | High mobility group box-1 protein |
| AAALAC | Association for Assessment and Accreditation of Laboratory Animal Care |
| IACUC | Institutional Animal Care and Use Committee |
| SPF | Specific pathogen Free |
| BIW | Twice a week |
| BID | Twice a day |
| BW | Body weight |
| EDTA | Ethylenediamine tetraacetic acid |
| GLP | Good Laboratory Practice for Non-clinical Laboratory Studies |
| *p.o.* | Oral gavage |
| *i.v.* | Intravenous administration |
| *i.p.* | Intraperitoneal injection administration |
| DPBS | Dulbecco's Phosphate Buffered Saline |
| DDH2O | Double distilled water |
| RT | Room temperature |
| SEM | Standard error of the mean |
| TGI | Tumor growth inhibition rate |
| TV | Tumor volume |
| TW | Tumor weight |
| FBS | Fetal bovine serum |
| PBS | Phosphate-buffered saline |
| MC | Methylcellulose |
| CO₂ | Carbon dioxide |
| CRT | Calreticulin |
| Annexin-V-FITC | Fluorescein isothiocyanate-labeled annexin |
| PI | Propidium iodide |

### Example 1: Study of the FAK target on the immunogenic cell death of Cobimetinib in melanoma cells

### Assay scheme:

### 1. Improving the sensitivity of Cobimetinib after FAK silencing

In the in vitro assay, different concentrations of Cobimetinib were given under the condition of silencing the FAK gene. In A375 cells, the cell killing curve was obtained and the IC50 value was determined. The cytotoxic effect of Cobimetinib in the control group and the FAK siRNA group was compared to explore the effect of the FAK target on the cytotoxicity of Cobimetinib.

The cell killing curve of Cobimetinib at different concentrations in melanoma cells A375 under the condition of FAK gene silencing was detected in the assay and the IC50 value was determined.

### 2. After FAK silencing, combination with Cobimetinib can effectively induce ICD in melanoma A375 cells

The effects of FAK silencing combined with Cobimetinib for 6 hours on the endoplasmic reticulum stress-related proteins GRP78 and Phospho-eIF2α (Ser51) were detected in the in vitro assay, as well as whether after 48 hours of the combination, the release of Calreticulin, the main target of ICD, from cells could be effectively increased.

### Assay materials:

FAK siRNA was provided by Gene Pharma, and the sequence is shown in the following table:
F: CCUGUAUGCCUAUCAGCUUTT;
R: AAGCUGAUAGGCAUACAGGTT
Cobimetinib was purchased from MCE, GDC-0973
Antibodies used in this assay:
Recombinant Alexa Fluor^{®} 647 fluorescent Anti-Calreticulin antibody (Abcam, ab196159), FAK antibody (CST, 3285S), GRP78 antibody (HUABIO, ER1706-50), Phospho-eIF2α (Ser51) (CST, 3398), eif2α (CST, 5324), HRP-labeled Alpha tubulin antibody (Proteintech, HRP -66031).
Laboratory apparatus:
Fluorescence microscopy (Olympus U-HGLGPS). Chemiluminescence imager (BIORAD chemidoc touch)

### Assay results:

### 1. IC50 curve of combination with cobimetinib against A375 after FAK silencing

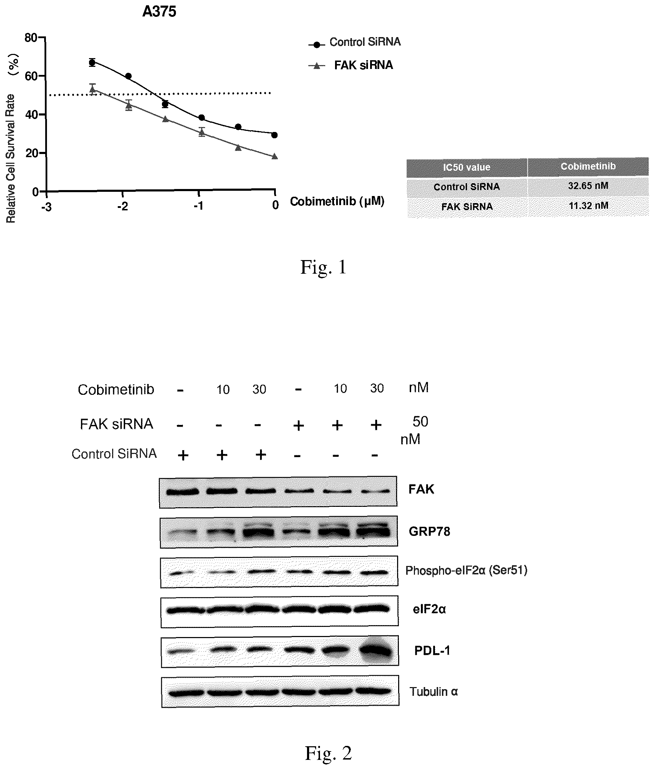

A 96-well cell plate was seeded with 5000 A375 cells/well, and two groups were transfected with control siRNA or FAK siRNA respectively. Cobimetinib was added 24 hours after the transfection. The highest concentration was 1 µM. A total of 6 concentrations were set for 3-fold gradient dilution. The lowest concentration of Cobimetinib was 4.115 nM. After the drug and cells were incubated for 72 hours, 10 µl CCK8 solution was added to each well and incubated at 37 degrees in a 5% carbon dioxide incubator for 2 hours. A microplate reader with the absorbance wavelength set to 450 nm was used for reading. The values read were compared to the DMSO control group and plotted using Graphpad 8.0. The results are shown in Fig. 1.

2. Combination with cobimetinib after FAK silencing significantly up-regulated the endoplasmic reticulum stress of A375

A375 cells were transfected with control siRNA or FAK siRNA at a final concentration of 50 nM. After 24 hours of transfection, different concentrations of Cobimetinib were given for 6 hours. The protein expression levels of FAK and endoplasmic reticulum stress-related proteins GRP78 and Phospho-eIF2α (phosphorylated-Phospho-eIF2α) (Ser51) were detected. The results are shown in Fig. 2.

3. Cobimetinib combined with IN10018 enhances the release and exposure of the ICD target calreticulin

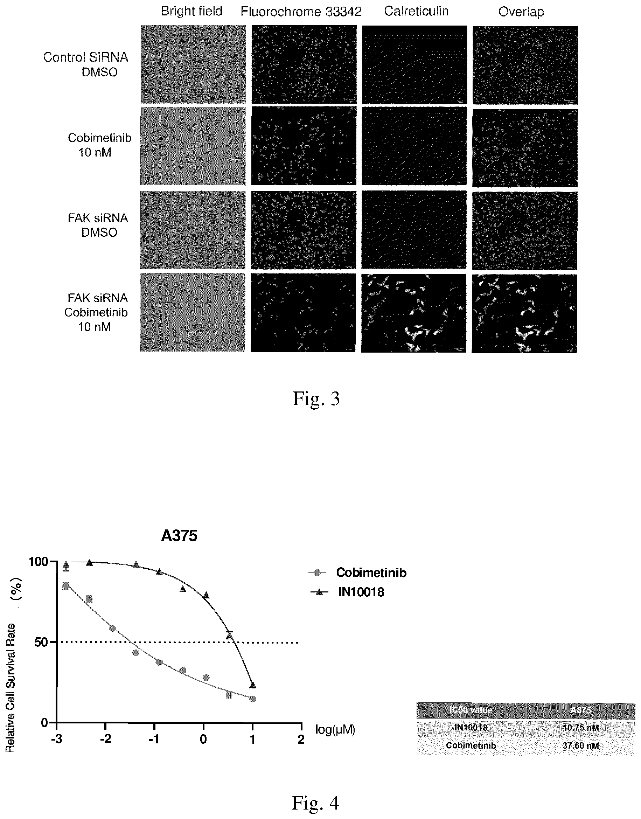

A375 cells were transfected with control siRNA or FAK siRNA at a final concentration of 50 nM. After 24 hours, A375 cells were treated with 10 nM Cobimetinib for 48 hours and then calreticulin was detected by immunofluorescence. The results are shown in Fig. 3.

### Assay conclusion:

Silencing FAK can significantly enhance the cell killing effect of cobimetinib on melanoma cells A375. This effect is related to the upregulation of endoplasmic reticulum stress. In the assay for detecting ICD markers, silencing FAK can significantly enhance the exposure and release of the DAMPs marker calreticulin, suggesting that silencing of FAK targets can effectively enhance immunogenic cell death in melanoma cells.

### Example 2: Study on IN10018 Enhanced Immunogenic Cell Death of Melanoma Cells for Cobimetinib

### Assay scheme:

### 1. IN10018 combined with Cobimetinib produces synergistic tumor killing effect

The cell killing curves of IN10018 and Cobimetinib in melanoma cell A375 was detected in the in vitro assay and the IC50 value was determined. The cytotoxic effects of cobimetinib at different doses combined with 3 µM IN10018 were explored.

### 2. IN10018 combined with Cobimetinib can effectively induce the ICD effect of melanoma cells A375

The effects of IN10018 and Cobimetinib on endoplasmic reticulum stress-related proteins GRP78, Phospho-eIF2α (Ser51), and DDIT3 after 6 hours of co-administration, and whether the release of ICD's main targets, Calreticulin and Annexin A1 (annexin A1), could be effectively enhanced by cells after 48 hours of co-administration were detected in the in vitro assay.

### Assay materials:

### 1) Drugs used in this assay

IN10018 was synthesized according to the method in patent WO2010058032. Cobimetinib was purchased from MCE GDC-0973

Antibodies used in this assay:
Recombinant Alexa Fluor^{®} 647 fluorescent Anti-Calreticulin antibody (Abcam, ab196159), Annexin A1 rabbit antibody (Cell signaling technology, 32934S), Hoechst33342 (Invitrogen, H21492), GRP78 antibody (HUABIO, ER1706-50), DDIT3 (HUABIO, ET1703-05), Phospho-eIF2α (Ser51) (CST, 3398), eif2α (CST, 5324), p44/42 MAPK (Erk1/2) (CST, 4695), Phospho-p44/42 MAPK (Erk1/2) (Thr202/Tyr204) (CST, 4370),
HRP-labeled Alpha tubulin antibody (Proteintech, HRP-66031).
Laboratory apparatus:
Fluorescence microscope (Olympus U-HGLGPS). Chemiluminescence imager (BIORAD chemidoc touch).

### Assay results:

### 1. IC50 curve of IN10018 and Cobimetinib monotherapy against A375

A 96-well cell plate was plated with 5000 A375 cells/well. After 24 hours, the drugs were added with a maximum concentration of 10 µM Cobimetinib and 30 µM IN10018, and a total of 9 concentrations were set in a 3-fold concentration gradient dilution. The lowest concentration of Cobimetinib was 1.52 nM and the lowest concentration of IN10018 was 4.57nM. After the drug and cells were incubated for 72 hours, 10 µL CCK8 solution was added to each well. After incubation in a 37-degree 5% carbon dioxide incubator for 2 hours, a microplate reader with the absorbance wavelength set to 450 nm was used for reading. The read values were compared with the DMSO control group and plotted using Graphpad 8.0. The results are shown in Fig. 4.

2. Synergistic killing effects of the combination of cobimetinib at different doses and 3 µM IN10018 on A375

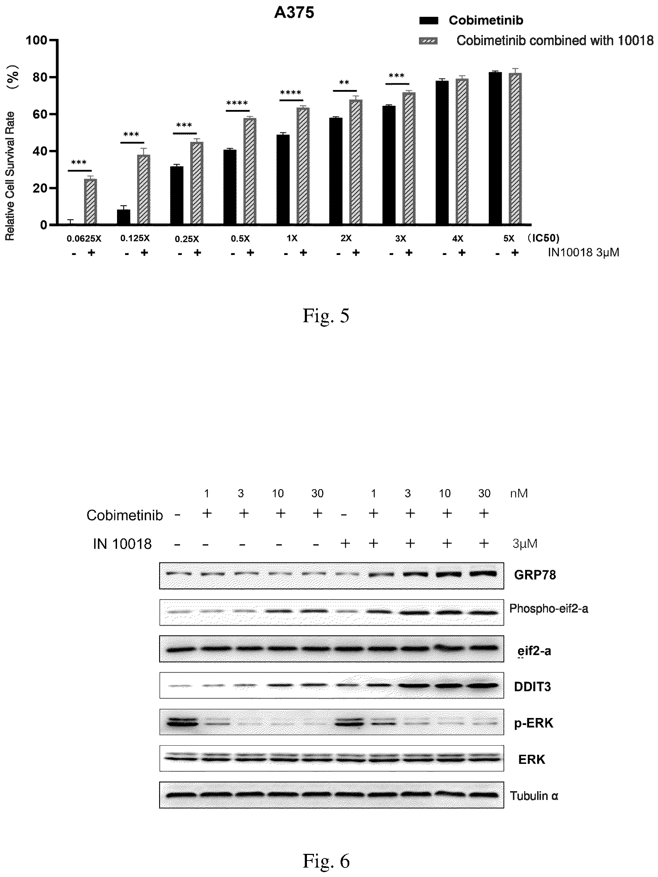

A 96-well cell plate was seeded with 5000 A375 cells/well. After 24 hours, the IC50 value of Cobimetinib was used as the initial point to set 4 sub-doses on the left and right sides. Cobimetinib was combined with 3 µM IN10018 at these doses. The cell viability was detected using CCK8 method after 72 hours. Graphpad8.0 was used for plotting. The results are shown in Fig. 5.

3. Cobimetinib combined with IN10018 significantly up-regulates endoplasmic reticulum stress in A375

A375 cells were treated with 1 nM, 3 nM, 10 nM and 30 nM Cobimetinib and 3 µM IN10018 respectively, and the protein level expression of endoplasmic reticulum stress-related proteins GRP78, Phospho-eIF2α (Ser51) and DDIT3 were detected 6 hours later. The results showed that compared with the monotherapy group, the expression levels of GRP78, Phospho-eIF2α (Ser51), and DDIT3 were significantly increased in the combination group, indicating that the endoplasmic reticulum stress was significantly increased in the combination of the two drugs (as shown in Fig. 6).

4. Cobimetinib combined with IN10018 enhances the release and exposure of ICD targets calreticulin and Annexin A1

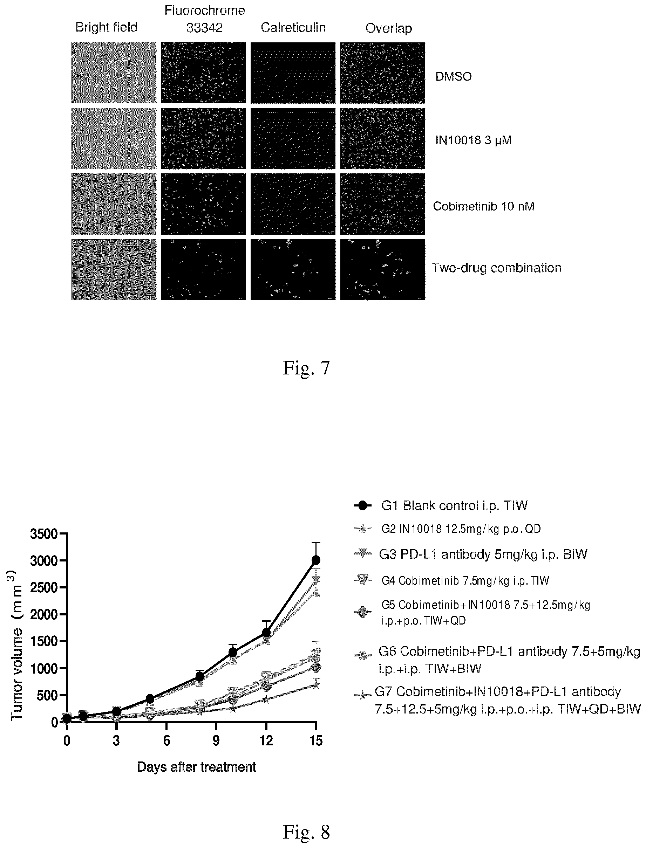

A375 cells were treated with 10 nM Cobimetinib and 3 µM IN10018. After 48 hours, calreticulin and Annexin A1 antibodies were used for fluorescent staining. The results show that compared with the monotherapy group, the release and exposure of Calreticulin and Annexin A1 were significantly enhanced in the combination group (as shown in Fig. 7).

### Assay results:

IN10018 can significantly enhance the cell killing effect of cobimetinib on melanoma cells A375. This effect is related to the upregulation of endoplasmic reticulum stress. In the assay for detecting ICD markers, it was found that IN10018 can significantly enhance the exposure and release of DAMPs markers, Calreticulin and Annexin A1, suggesting that IN10018 can effectively enhance the immunogenic cell death of melanoma cells.

### Example 3: In vivo antitumor efficacy study of Cobimetinib in a subcutaneous allograft tumor model of colon cancer CT-26 cell in BALB/c mouse

### Assay materials:

Mice: Female BALB/c mice aged 6-8 weeks were purchased from Beijing Weitong Lihua Laboratory Animal Co., Ltd. The assay began after the animals arrived and were adapted to the experimental environment. The animals were kept in IVC (independent ventilation system) cages (6 per cage) in an SPF-level animal room. All cages, bedding and drinking water must be sterilized before use. All experimenters should wear protective clothing and latex gloves when operating in the animal room. The animal information cards for each cage should indicate the number of animals in the cage, gender, strain, receipt date, dosing regimen, assay number, group and start date of the assay. Cages, feed and drinking water were changed twice a week. The breeding environment and lighting conditions are as follows:
Temperature: 20-26°C; Humidity: 40-70%; illumination period: 12 hours of light, 12 hours of dark
Cages: Made of polycarbonate, volume: 300 mm × 180 mm × 150 mm. Bedding is corn cobs, changed twice a week.
Food: The assay animals had free access to food (irradiated sterilized, dry pelleted food) throughout the assay period.
Drinking water: Assay animals were free to drink sterile water.
Cage identification: The animal information cards for each cage should indicate the number of animals in the cage, gender, strain, receipt date, dosing regimen, assay number, group and start date of the assay.
Animal identification: Assay animals were identified with ear tags.

The compound information is shown in Table 1.

**Table 1. Compound information**

| **Drug Name** | **Concentration (mg/mL)** | **Storage conditions** | **Source** | **vehicle** | **Total** |
|---|---|---|---|---|---|
| Cobimetinib | / | Normal temperature | MCE (HY-13064) | 10%DMSO+ 40%PEG300+ 5%Tween-80+45%PBS | 70mg |
| IN10018 | / | 4 °C | Synthesized according to the method in patent WO2010058032 | DPBS | 160 mg |
| PDL1 Antibody | 8.13 mg/mL | 4 °C | Bioxcell (BE0101) | DPBS | 20 mg |
| Polyethylene glycol 300 (PEG300) | / | Normal temperature | Sinopharm Group (30150728) | / | / |
| Dimethyl sulfoxide (DMSO) | / | Normal temperature | SIGMA (D2660) | / | / |
| Tween 80 (Tween-80) | / | Normal temperature | Aladdin (T104866) | / | / |

Colorectal cancer cell CT-26 (source: Nanjing Kebai Biotechnology Co., Ltd., catalog number: CBP60043) was maintained and sub-cultured by Inxmed Biotechnology (Nanjing) Co., Ltd. The cells were cultured in a monolayer in vitro, and the culture conditions were RPMI-1640 medium with 10 % fetal bovine serum, and cultured in a 37 °C 5% CO₂ incubator. Two to three times a week, routine digestion and subculture were performed with trypsin -EDTA. When the cells were in the exponential growth phase and the saturation was 80%-90%, the cells were harvested, counted, and inoculated.

### Cell seeding and grouping

0.1 mL of cell suspension containing 3×10⁵ cells was subcutaneously inoculated on the right back of each mouse. When the tumor volume reached ~58 mm³ (10 days after cell inoculation), the mice were randomly divided into groups for drug administration according to the tumor volume. The group information is shown in Table 2.

**Table 2. Dosing regimen of the test substance in the CT-26 mouse transplant tumor model**

| **Group** | **Compound treatment** | **Number of animals¹** | **Dosage (mg/kg)** | **Dosing volume parameters (mL/kg)²** | **Route of administration** | **Dosing frequency** |
|---|---|---|---|---|---|---|
| 1 | Control group | 6 | N/A | 10 | *ip* | TIW |
| 2 | IN10018 | 6 | 12.5 | 10 | *po* | QD * 2 weeks |
| 3 | PDL 1 Antibody | 6 | 5 | 10 | *ip* | BIW * 2 weeks |
| 4 | Cobimetinib | 6 | 7.5 | 10 | *ip* | T IW*31 days |
| 5 | Cobimetinib + IN10018 | 6 | 7.5+ 12.5 | 10 | *ip + po* | TI W*31 days +QD *31 days |
| 6 | Cobimetinib + PDL1 antibody | 6 | 7.5+ 5 | 10 | *ip+ip* | TI W*31 days +BIW *31 days |
| 7 | Cobimetinib + IN10018 + PDL1 antibody | 6 | 7.5+12.5 +5 | 10 | *ip + po + ip* | TI W*31 days +QD *31 days +BIW *31 days |

| | | | | | | |
|---|---|---|---|---|---|---|
| Note: 1. Number of mice in each group; 2. Dosing volume: 10 mL/kg based on mouse body weight. If the body weight drops by more than 15%, stop dosing the animal; resume dosing when the body weight recovers to a decrease of 10%. | | | | | | |

The preparation of the test substances is shown in Table 3

**Table 3. Test substance configuration method**

| **Drug Name** | **Dosage (mg/kg)** | **Concentr ation (mg/mL)** | **Preparation process** |
|---|---|---|---|
| **Control group** (10% DMSO + 40% PEG300 + 5% Tween-80 + 45% PBS) | / | / | 20 ml of DMSO was aspirated, 80 ml of PEG300 was added, mixed well, then 10 ml of Tween-80 was added, mixed well again, and finally 90 ml of PBS was added and mixed well. |
| **Cobimetinib** | 7.5 | 0.75 | A bottle of 50mg Cobimetinib was taken, and 6.666ml DMSO was added to prepare a mother solution. Every time of using, 0.6ml of the above mother solution was taken, 2.4ml PEG300 was added, mixed well, then 0.3ml Tween-80 was added, mixed well, and finally 2.7ml PBS was added, mixed well. Prepared and used immediately. |
| **IN10018** | 12.5 | 1.25 | 27 mg of IN10018 was weighed, 21.6 ml of PBS was added, mixed well, and prepared once every 4 days. |
| **PDL1 Antibody** | 5.00 | 0.50 | 0.332 mL of PDL1 antibody (8.13 mg/mL) was pipetted, diluted with 5.068 mL of DPBS, mixed well to make a clear solution, which was used immediately. |

### Daily observation of assay animals

The formulation of this experimental plan and any modification have been evaluated and approved by Yunqiao Biotechnology's IACUC. The use and welfare of experimental animals were carried out in accordance with AAALAC regulations. The health status and death of animals were monitored every day. Routine examination includes observing the effects of tumor growth and drug treatment on the daily behavior of animals, such as behavioral activities, food and water intake (visual observation only), body weight change, appearance signs or other abnormal conditions. Based on the number of animals in each group, the death number and side effects of animals in the group were recorded.

### Assay terminating

If the animal's health continues to deteriorate, or the tumor volume exceeds 2500 mm³, or the animal has a serious disease or pain, the animal must be euthanized. The veterinarian should be notified and the animal should be euthanized if the following situations occur: obvious emaciation, weight loss greater than 20%; unable to eat and drink freely; the average tumor volume in the control group reaches 2500 mm³, and the assay is terminated; the animal exhibits the following clinical manifestations and continues to deteriorate: standing hair, arching back, the color of ears, nose, eyes or feet turns to white, shortness of breath, convulsions, continuous diarrhea, dehydration, slow movement and vocalization.

### Tumor measurement and assay index

The diameter of tumor was measured with vernier caliper, 3 times a week. The formula for calculating the tumor volume is: V = 0.5 × a × b², where a and b represent the long and short diameters of the tumor respectively.

Referring to the tumor volume on the first day after grouping, the tumor growth inhibition rate TGI (%) was calculated according to the following formula: TGI (%) = [1-(average tumor volume of a certain dosing group - average tumor volume at the beginning of the dosing group)/(average tumor volume of the vehicle control group-average tumor volume at the beginning of treatment of the vehicle control group)] × 100%.

Two weeks after administration, animals in groups G1, G2 and G3 were euthanized due to excessive tumor volume. The remaining groups continued to receive the drug and the survival rate of the animals was observed. Animals were considered dead when they died naturally or when the tumor volume was greater than 2500 mm³. The survival rate and life cycle of the animals after administration were calculated based on this.

### Statistical analysis

Statistical analysis was performed based on tumor volume and tumor weight at the end of the trial using Prism Graphpad software. Comparisons between multiple groups were analyzed using the Two-way ANOVA test, and P < 0.05 was considered as having significant differences.

The survival rate of animals was analyzed using the Gehan-Breslow-Wilcoxon test, and P < 0.05 was considered as having significant difference.

### Assay results:

The in vivo efficacy of the test substance Cobimetinib and/or PDL1 antibody combined with IN10018 in the CT-26 mouse colorectal cancer cell subcutaneous allograft tumor model of BALB/c mice was tested. After cell inoculation, the tumor growth was observed every day, and the mice were grouped according to the tumor volume on the 10th day after inoculation. The average tumor volume at the entry was about 58mm³. Due to tumor burden, the mice in the control group and each monotherapy group (except Cobimetinib 5mpk group) were euthanized on the 25th day after inoculation, that is, the 15th day after group administration. The survival cycle of animals in the remaining groups continued to be observed until the survival rate of the last group dropped below 50%, and all assays were terminated.

On the 15th day after group administration, the tumor volume of the control group was 3007.0 ± 799.4 mm³. The tumor volumes of treatment groups IN10018 (12.5 mg/kg) monotherapy, PDL1 antibody (5 mg/kg), Cobimetinib (7.5 mg/kg) monotherapy, Cobimetinib + IN 100 18 (7.5 + 12.5 mg/kg), Cobimetinib + PDL1 antibody (7.5 + 5 mg/kg) and Cobimetinib + IN10018 + PDL1 antibody (7.5 +12.5 + 5 mg/kg) were 2418.8 ± 436.5 mm³, 2614.2 ± 568.4 mm³, 1261.3 ± 565.4 mm³, 1016.5 ± 271.3 mm³, 1196.9 ± 411.5 mm³ and 686.3 ± 292.9 mm³, respectively, see Table 4 for details. The comprehensive tumor volume was compared with that of the control group. The tumor inhibition rates TGI of IN10018 (12.5 mpk) monotherapy, PDL1 antibody (5 mg/kg), Cobimetinib (7.5 mg/kg) monotherapy, Cobimetinib + IN10018 (7.5 + 12.5 mg/kg), Cobimetinib + PDL1 antibody (7.5 + 5 mg /kg) and Cobimetinib + IN10018 + PDL1 antibody (7.5 +12.5 + 5 mg/kg) were 19.9 % (*p* = 0.0002), 13.3 % (*p* = 0.0241), 59.2 % (*p <* 0.0001), 67.5 % (*p* < 0.0001), 61.4 % (*p* < 0.0001) and 78.7 % (*p* < 0.0001), respectively. The comprehensive tumor volume was compared with the Cobimetinib + IN10018 + PDL1 antibody (7.5 +12.5 + 5 mg/kg) group for statistical analysis. The P values of the control group, IN10018 (12.5mg/kg) monotherapy, PDL1 antibody (5 mg/kg), Cobimetinib (7.5mpk) monotherapy, Cobimetinib + IN10018 (7.5 + 12.5 mg/kg) and Cobimetinib + PDL1 antibody (7.5 + 5 mg/kg) groups were *p* < 0.0001, *p* < 0.0001, *p* < 0.0001, *p* = 0.0002, *p* = 0.0805 and *p* = 0.0015, respectively, see Table 4 for details. The tumor volumes of each dose group at different time periods are shown in Fig. 8.

**Table 4. Evaluation of the anti-tumor effect of the test substance on the BALB/c mouse transplant tumor model of mouse colon cancer CT-26 cells (based on the data on the 15th day after group administration)**

| **Group** | **Tumor volume on day 0 (mm ³) ¹** | **Tumor volume on day 15 (mm ³)** | **TGI (%)** | **P value ²** | **P value ³** |
|---|---|---|---|---|---|
| **Control group** | 58.5 ± 15.5 | 3007.0 ± 799.4 | -- | | < 0.0001**** |
| **IN10018 monotherapy** | 56.1 ± 10.0 | 2418.8 ± 436.5 | 19.9 | 0.0002*** | < 0.0001**** |
| **PDL1 Antibody** | 57.0 ± 14.2 | 2614.2 ± 568.4 | 13.3 | 0.0241* | < 0.0001**** |
| **Cobimetinib monotherapy** | 58.2 ± 12.9 | 1261.3 ± 565.4 | 59.2 | < 0.0001**** | 0.0002 *** |
| **Cobimetinib + IN10018** | 57.9 ± 12.7 | 1016.5 ± 271.3 | 67.5 | < 0.0001**** | 0.0805 |
| **Cobimetinib + PDL1 antibody** | 57.5 ± 12.0 | 1196.9 ± 411.5 | 61.4 | < 0.0001**** | 0.0015 ** |
| **Cobimetinib + IN10018 + PDL1 antibody** | 59.1 ± 15.6 | 686.3 ± 292.9 | 78.7 | < 0.0001**** | -- |

| | | | | | |
|---|---|---|---|---|---|
| Note: 1. Calculated based on the number of days after group administration, data are mean ± standard error. 2. *: p<0.05, ****: p<0.0001, vs. control group, Two-way ANOVA. 3. **: p<0.01, ***: p<0.001, ****: p<0.0001, vs. Cobimetinib+IN10018+PDL1 antibody (7.5+12.5+5 mg/kg) group, Two-way ANOVA. | | | | | |

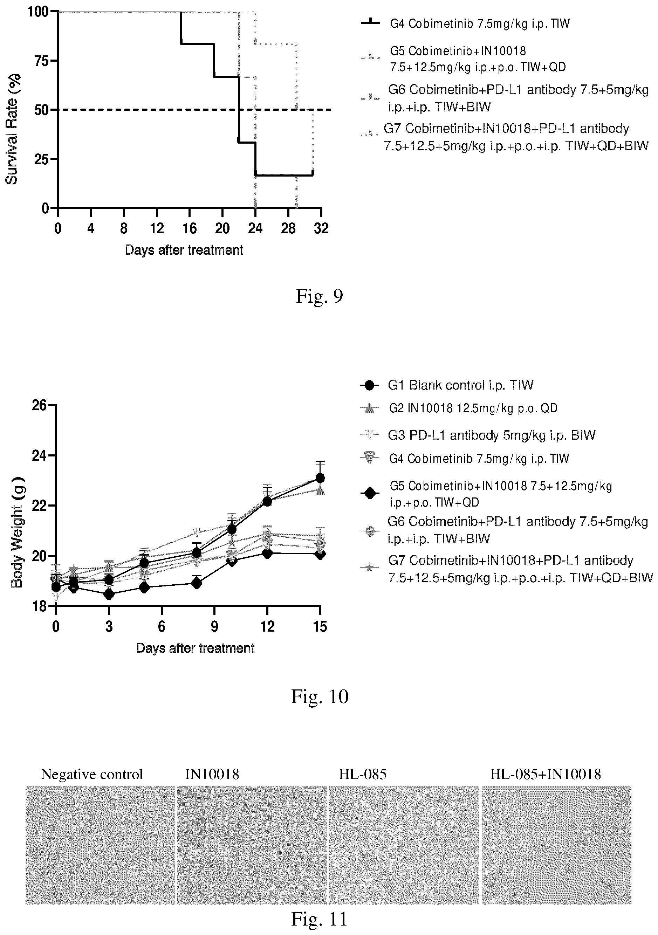

After 15 days of administration, the control group, IN10018 (12.5 mg/kg) monotherapy, PDL1 antibody (5 mg/kg) groups were euthanized due to excessive tumor volume. The dosing of the remaining Cobimetinib (7.5 mg/kg) monotherapy, Cobimetinib + IN10018 (7.5 + 12.5 mg/kg), Cobimetinib + PDL1 antibody (7.5 + 5 mg/kg) and Cobimetinib + IN10018 + PDL1 antibody (7.5 + 12.5 + 5 mg/kg) groups were stopped, and continued to observe and record tumor volume. Tumor volume greater than 2500mm³ or natural death can be recorded as animal death, and the changes in survival rate are observed until the survival rate of the group with the best survival rate drops below 50%. As of the 31st day after administration, the group with the best survival rate was the Cobimetinib + IN10018 + PDL1 antibody (7.5 +12.5+ 5 mg /kg) group, with a median survival time and survival rate of 30 days and 16.7%, respectively. The median survival time and survival rate of the remaining Cobimetinib (7.5 mg/kg) monotherapy, Cobimetinib + IN10018 (7.5+ 12.5 mg/kg) and Cobimetinib + PDL1 antibody (7.5+ 5 mg/kg) groups were 22 days & 16.7%, 24 days & 0%, and 22 days & 0%, respectively. The Gehan-Breslow-Wilcoxon test was used for statistical analysis. Compared with Cobimetinib + IN10018+ PDL1 antibody (7.5+12.5+5mg/kg) group, the P values of the cobimetinib + PDL1 antibody (7.5 +12.5+ 5 mg/kg) group, cobimetinib (7.5 mg/kg) monotherapy, cobimetinib + IN10018 (7.5+ 12.5 mg/kg) and cobimetinib + PDL1 antibody (7.5+ 5 mg / kg) groups were p = 0.0434, p = 0.0137 and p = 0.0029, respectively. See Table 5 for details. The survival rate of each dose group at different time periods is shown in Fig. 9.

**Table 5. Evaluation of the tumor survival rate of the test substance in the BALB/c mouse transplant tumor model of mouse colon cancer CT-26 cells (based on the data on the 31st day after group administration)**

| **Group** | **Survival rate on Day 0 (%) ¹** | **Survival rate on Day 31 (%)** | **Median survival time (days)** | **P value²** |
|---|---|---|---|---|
| **Cobimetinib monotherapy** | 100 | 16.7 | 22 | 0.0434 * |
| **Cobimetinib + IN10018** | 100 | 0 | 24 | 0.0137 * |
| **Cobimetinib + PDL1 antibody** | 100 | 0 | 22 | 0.0029 ** |
| **Cobimetinib + IN10018 + PDL1 antibody** | 100 | 16.7 | 30 | -- |

| | | | | |
|---|---|---|---|---|
| Note: 1. Calculated according to the number of days after group administration, survival rate = the number of surviving animals/6*100. 2. *: p<0.05, **: p<0.01, vs. Cobimetinib + IN10018 + PDL1 antibody (7.5+12.5+5 mg/kg) group, Gehan-Breslow-Wilcoxon test | | | | |

The assay was carried out according to the dosing regimen. During the assay, the animals' activities such as food and water intakes were observed every day, and the weight of the animals was recorded three times a week. The animal weight curve is shown in Fig. 9. During the entire dosing period, the weight of the animals in each group did not decrease significantly and were in good condition.

### Assay conclusion:

Compared with the blank control group, each dosing group had a significant tumor growth inhibitory effect, and there were statistical differences compared with the control group. The average tumor volume of the three-drug combination group of Cobimetinib + IN10018 + PDL1 antibody (7.5+12.5+5 mg/kg) was the smallest, and except for the Cobimetinib + IN10018 (7.5+ 12.5 mg/kg) group, the other groups were statistically different from the three-drug combination group. Although the Cobimetinib + IN10018 (7.5+ 12.5 mg/kg) group had no statistical difference from the Cobimetinib + IN10018 + PDL1 antibody (7.5+12.5+5 mg/kg) three-drug combination group, the average tumor volume was also greater than that of the three-drug combination group. The above data shows that the three-drug combination group has better efficacy than the monotherapy group and the two-drug combination group.

Judging from the survival rate data, the three-drug combination group of Cobimetinib + IN10018 + PDL1 antibody (7.5+12.5+5 mg/kg) had better survival rates and median survival time than Cobimetinib (7.5m g/kg) monotherapy and Cobimetinib + IN 100 18 (7.5+ 12.5 mg/kg) and Cobimetinib + PDL1 antibody (7.5+ 5 mg /kg) groups, and was statistically different from these three groups. The above data shows that the three-drug combination of Cobimetinib + IN10018 + PDL1 antibody (7.5+12.5+5mg/kg) can significantly reduce the death of animals.

There was no significant weight loss in the animals in each group and they were in good condition, indicating that the animals tolerated the administration of Cobimetinib + IN10018 + PDL1 antibody (7.5+12.5+5mg/kg).

### Example 4: In vitro efficacy study of IN10018 and HL-085 in colon cancer CT-26 cell line

### Assay materials:

### 1) Drugs used in this assay

HL-085 was provided by Shanghai Kezhou Pharmaceutical Research and Development Co., Ltd.

### 2) Antibodies used in this assay

Recombinant Alexa Fluor^{®} 647 fluorescent anti-calreticulin antibody (Abcam, Cat No.: ab196159, Lot No.: CR33676773). Annexin-Modulation Assay Kit (Beyotime, Cat No.: Beyotime, Lot No.: 021921210811).

### Assay method:

CT26 cells were cultured with RPMI 1640 (Shanghai Yuanpei, Cat No.: L210KJ, Lot No.: F210916) + 10% FBS (Gibco, Cat No.: 10099-141c, Lot No.: 2158737cp) and passaged twice. When the cells were in good condition, the culture plates were placed in 24-well plates. After 24 hours of cell spreading, four groups were set up. The first group was the control group, to which culture medium was added, the second group was IN10018 at a concentration of 5 µM, the third group was HL-085 at a concentration of 1 µM, and the fourth group was IN10018 (5 µM) combined with HL-085(1 µM). The drugs were mixed and cultured at 37°C in a 5% CO₂ incubator for 48 hours.

### Assay results

After 48 hours of drug action, cells were observed under a microscope and photographed to confirm the cell killing effect. Then the cells were collected for flow analysis, and the cells were washed twice with flow buffer (PBS + 2% FBS) and 0.5 µl AF647. Anti-calreticulin antibody (abcam) was added to each well and mixed. The mixture was incubated at 4 °C in the dark, flow buffer was added after 20 min of incubation, the annexin staining kit (Beyotime) was used and 195 µl Annexin-V-FITC conjugate was added. After pipetting and mixing with the cells, 5 µl Annexin-V-FITC antibody was added and gently mixed. Finally, 10 µl PI dye was added and mixed. The mixture was incubated at room temperature in the dark for 15 min, and the samples were sent to the flow cytometer for signal measurement.

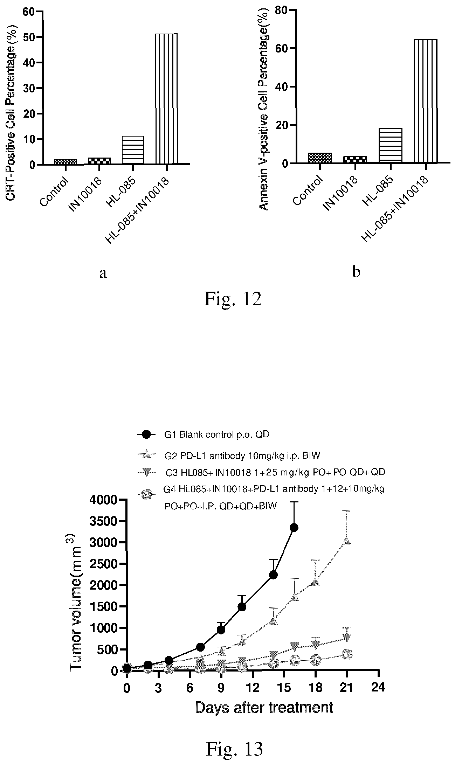

By observing the cells under a microscope, the cell status of the HL-085 monotherapy group and the two-drug combination group was poor. The cell status of the two-drug combination group was the worst, and there was more cell death. This shows that the HL-085 monotherapy group and the two-drug combination group have cell killing effects. The two-drug combination group had the most significant cell killing effect, while the cell status in the control group and IN10018 group was better, as shown in Fig. 11. Flow cytometry analysis results show that the CRT positive rate and Annexin-V positive rate of the two-drug combination group were significantly better than those of the monotherapy group and the control group, see Fig. 12a and Fig. 12b.

### Example 5: Study on the in vivo anti-tumor efficacy of HL085 in colon cancer CT-26 cell BALB/c mouse subcutaneous allograft tumor model

### Assay materials:

Mice: Female BALB/c mice aged 6-8 weeks were purchased from Beijing Weitong Lihua Laboratory Animal Co., Ltd. The assay began after the animals arrived and were adapted to the assay environment. The animals were kept in IVC (independent ventilation system) cages (5 per cage) in an SPF-level animal room. All cages, bedding and drinking water must be sterilized before use. All experimenters should wear protective clothing and latex gloves when operating in the animal room. The animal information cards for each cage should indicate the number of animals in the cage, gender, strain, receipt date, dosing regimen, assay number, group and start date of the assay. Cages, feed and drinking water were changed twice a week. The breeding environment and lighting conditions are as follows:
Temperature: 20-26 °C; Humidity: 40-70%; illumination period: 12 hours of light, 12 hours of dark.
Cages: Made of polycarbonate, volume: 300 mm × 180 mm × 150 mm. Bedding is corn cobs, changed twice a week.
Food: The assay animals had free access to food (irradiated sterilized, dry pelleted food) throughout the assay period.
Drinking water: Assay animals were free to drink sterile water.
Cage identification: The animal information cards for each cage should indicate the number of animals in the cage, gender, strain, receipt date, dosing regimen, assay number, group and start date of the assay.
Animal identification: Assay animals were identified with ear tags.

The compound information is shown in Table 6:

**Table 6. Compound information**

| **Drug Name** | **Concentration (mg/mL)** | **Storage conditions** | **Source** | **Vehicle** | **Total** |
|---|---|---|---|---|---|
| HL085 | / | Normal temperatur e | Shanghai Kezhou | 0.5%HPMC+ 0.2%Tween-80 | 9.5 mg |
| IN10018 | / | 4 °C | Synthesized according to the method in patent WO2010058032 | DPBS | 235 mg |
| PDL1 Antibody | 8.13 mg/mL | 4 °C | Bioxcell (BE0101) | DPBS | 25 mg |
| Hydroxypropyl methylcellulos e (HPMC) | / | Normal temperatur e | Aladdin (H108817) | / | / |
| Tween-80 | / | Normal temperatur e | Aladdin (T104866) | / | / |

Colorectal cancer cell CT-26 (source: Nanjing Kebai Biotechnology Co., Ltd., catalog number: CBP60043) was maintained and sub-cultured by Inxmed Biotechnology (Nanjing) Co., Ltd. The cells were cultured in a monolayer in vitro, and the culture conditions were RPMI- 1640 medium plus 10% fetal bovine serum, and cultured in a 37 °C 5% CO₂ incubator.The cells were routinely digested and sub-cultured with trypsin-EDTA two to three times a week. When the cells were in the exponential growth phase and the saturation was 80%-90%, the cells were harvested, counted, and inoculated.

### Cell seeding and grouping

0.1 mL of cell suspension containing 3× 10⁵ cells was subcutaneously inoculated on the right back of each mouse. When the tumor volume reached ~59 mm³ (12 days after cell inoculation), the mice were randomly divided into groups for drug administration according to the tumor volume. The group information is shown in Table 7.

**Table 7. Dosing regimen of the test substance in the CT-26 mouse transplant tumor model**

| **Group** | **Compound treatment** | **Animal number¹** | **Dosage (mg/kg)** | **Dosing volume parameters (mL/kg) ²** | **Route of administration** | **Dosing frequency** |
|---|---|---|---|---|---|---|
| 1 | Control group | 7 | N/A | 10 | *po* | QD |
| 2 | PDL1 Antibody | 7 | 10 | 10 | *ip* | BIW* 3 weeks |
| 5 | HL - 085 + IN10018 | 7 | 1+ 25 | 10 | *po+po* | Q D * 3 weeks + Q D * 3 weeks |
| 6 | HL - 085 + IN10018 + PDL1 antibody | 7 | 1+25 + 10 | 10 | *po + po* + *ip* | Q D * 3 weeks + Q D * 3 weeks + BIW* 3 weeks |

| | | | | | | |
|---|---|---|---|---|---|---|
| Note: 1. Number of mice in each group; 2. Dosing volume: 10 mL/kg based on mouse body weight. If the body weight drops by more than 15%, stop dosing the animal; resume dosing when the body weight recovers to a decrease of 10%. | | | | | | |

### Preparation of test substance

### See Table 8 for details

**Table 8. Test substance configuration method**

| **Drug Name** | **Dose (mg/kg)** | **Concentration (mg/mL)** | **Preparation process** |
|---|---|---|---|
| **Control group** (0.5% HPMC + 0.2% Tween-80) | / | / | 2.5 g HPMC was weighed, 400 ml DDH₂O was added, mixed by ultrasonic vortexing, then 1 ml Tween-80 was added and vortexed to obtain a clear solution, and finally the volume was adjusted to 500 ml. |
| **HL085** | 1 | 0.1 | 1.6 mg HL085 was weighed, 16.0 mL of the control group solution was added, mixed by ultrasonic vibration, to obtain a suspension, which was prepared once every 4 days. |
| **IN10018** | 25 | 2.5 | 42 mg IN10018 was pipetted, diluted with 16.8 mL DPBS, mixed well to make a clear solution, which was prepared every 4 days. |
| **PDL1 Antibody** | 10.00 | 1.00 | 0.517 mL of PDL1 antibody (8.13 mg/mL) was pipetted, diluted with 3.683 mL of DPBS, mixed thoroughly to make a clear solution, which was used immediately. |

### Daily observation of assay animals

The determination of this experimental plan and any modification have been evaluated and approved by Yunqiao Biotechnology's IACUC. The use and welfare of assay animals were carried out in accordance with AAALAC regulations. The health status and mortality of animals were monitored daily. Routine examinations include observing the effects of tumor growth and drug treatment on the daily behavior of animals, such as behavioral activities, food and water intake (only visual observation), weight changes, physical signs or other abnormal conditions. The number of deaths and side effects of animals in each group were recorded based on the number of animals in each group.

### Assay terminating

If the animal's health continues to deteriorate, or the tumor volume exceeds 2500 mm³, or the animal has a serious disease or pain, the animal must be euthanized. The veterinarian should be notified and the animal should be euthanized if the following situations occur: obvious emaciation, weight loss greater than 20%; unable to eat and drink freely; the average tumor volume in the control group reaches 2500 mm³, and the assay is terminated; the animal exhibits the following clinical manifestations and continues to deteriorate: standing hair, arching back, the color of ears, nose, eyes or feet turns to white, shortness of breath, convulsions, continuous diarrhea, dehydration, slow movement and vocalization.

### Tumor measurements and assay parameters

Tumor diameter was measured with a vernier caliper three times a week. The tumor volume was calculated using the formula: V = 0.5 × *a* × *b²,* where *a* and *b* represent the long and short diameters of the tumor, respectively.

Referring to the tumor volume on the first day after grouping, the tumor growth inhibition rate TGI (%) was calculated according to the following formula. TGI (%) =[1-(Average tumor volume of a certain dosing group-Average tumor volume of the dosing group at the beginning)/(Average tumor volume of the vehicle control group-Average tumor volume of the vehicle control group at the beginning of treatment)]× 100%.

### Statistical analysis

Statistical analysis was based on tumor volume and tumor weight at the end of the trial using Prism Graphpad software. Comparisons between multiple groups were analyzed using Two- way ANOVA test. *P* < 0.05 was considered as having significant difference.

### Assay results

The in vivo efficacy of the test substances HL085 and/or IN10018 combined with PDL1 antibody in the CT-26 mouse colorectal cancer cell subcutaneous allograft tumor model of BALB/c mice was tested. After cell inoculation, tumor growth was observed every day, and the mice were grouped according to tumor volume on the 12th day after inoculation. The average tumor volume at the entry was about 59 mm³. Due to tumor burden, the mice in the control group were euthanized on the 28th day after inoculation, that is, the 16th day after group administration. All other mice continued to be administered and were euthanized on the 33rd day after inoculation, that is, the 21st day after group administration, and the entire assay was terminated.

On the 16th day after group administration, the tumor volume of the control group was 3330.1 ± 1587.5 mm³. The tumor volumes of PDL1 antibody (10 mg/kg), HL085 + IN10018 (1+25 mg/kg) and HL085 + IN10018 + PDL1 antibody (1 + 25 + 10 mg/kg) treatment groups were 1723.0 ± 1122.8 mm³, 522.3 ± 327.3 mm³ and 228.4 ± 102.2 mm³, respectively, as shown in Table 9. The comprehensive tumor volume was compared with that of the control group. The tumor inhibition rates TGI of PDL1 antibody (10 mg/kg), HL085 + IN10018 (1+25mg/kg), and HL085 + IN10018 + PDL1 antibody (1+25+10 mg/kg) groups were 49.2 % (*p* < 0.0001), 85.8 % (*p* < 0.0001) and 94.8 % (*p* < 0.0001), respectively. See Table 9 for details. The tumor volumes of each dose group at different time periods are shown in Fig. 13.

After 16 days of administration, the mice in the control group were euthanized due to excessive tumor volume, and the administration of the remaining groups PDL1 antibody (10 mg/kg), HL085 + IN10018 (1+25mg/kg) and HL085 + IN10018 + PDL1 antibody (1 + 25 + 10 mg/kg) was continued, and the tumor volumes were continuously observed and recorded until the 21st day after dosing, where the tumor volumes were 3038.0 ± 1798.7 mm³, 737.0 ± 658.5 mm³, and 360.9 ± 161.3 mm³, respectively. The comprehensive tumor volume was compared with that of the HL085 + IN10018 + PDL1 antibody (1 + 25 + 10 mg / kg) group for statistical analysis. The P values of PDL1 antibody (10 mg/kg) and IN10018 (1+25 mg/kg) were: *p <* 0.0001 and *p* = 0.0461, see Table 9 for details. The tumor volumes of each dose group at different time periods are shown in Fig. 13.

**Table 9 Evaluation of the tumor inhibition effect of the test substance on the BALB/c mouse transplant tumor model of mouse colon cancer CT-26 cells (based on the data on the 16th and 21st days after group administration)**

| **Group** | **Tumor volume on Day 0 (mm³) ¹** | **Tumor volume on day 16 (mm³)** | **TGI (%)** | **P value²** | **Tumor volume on Day 21 (mm³)** | **P value³** |
|---|---|---|---|---|---|---|
| **Control group** | 58.6 ± 16.6 | 3330.1 ± 1587.5 | / | / | / | / |
| **PDL1 Antibody** | 59.5 ± 18.0 | 1723.0 ± 1122.8 | 49.2 | <0.0001**** | 3038.0 ± 1798.7 | <0.0001*** * |
| **HL085 + IN10018** | 58.5 ± 18.2 | 522.3 ± 327.3 | 85.8 | <0.0001**** | 737.0 ± 658.5 | *p* = 0.0461 * |
| **HL085 + IN10018 + PDL1 Antibody** | 58.9 ± 18.2 | 228.4 ± 102.2 | 94.8 | <0.0001**** | 360.9 ± 161.3 | / |

| | | | | | | |
|---|---|---|---|---|---|---|
| Note: 1. Calculated based on the number of days after group administration, data are mean ± standard error. 2. ****: p<0.0001, vs. control group, Two-way ANOVA. 3. *: p<0.05, ****: p<0.0001, vs. HL085+IN10018+PDL1 antibody (1+25+10 mg/kg) group, Two-way ANOVA. | | | | | | |

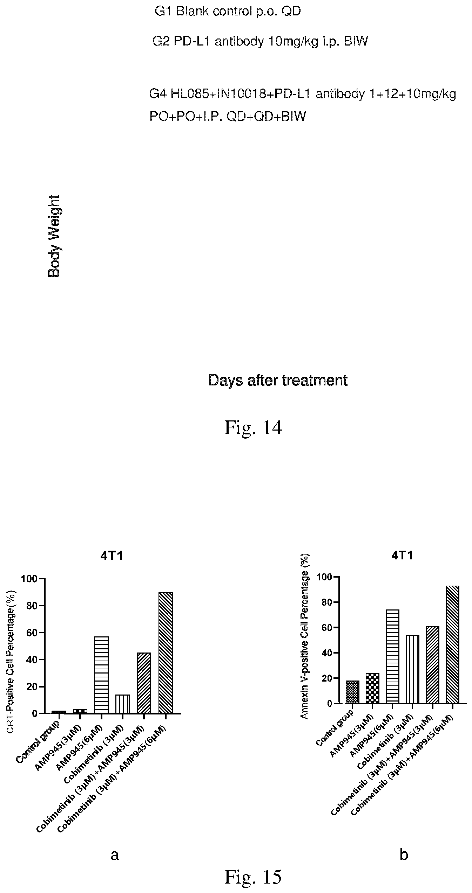

The assay was carried out according to the dosing regimen. During the assay, the animals' activities such as food and drink intakes were observed every day, and the weight of the animals was recorded three times a week. The animal weight curve is shown in Fig. 14. During the entire dosing period, the weight of the animals in each group did not decrease significantly and they were in good condition.

### Conclusion

Compared with the blank control group, PD-L1 antibody (10 mg/kg), HL085 + IN10018 (1+25 mg/kg) and HL085 + IN10018 + PDL1 antibody (1 + 2 5+ 10 mg / kg) groups show significant tumor growth inhibition, with statistically significant differences compared to the control group. Comprehensively considering the entire dosing cycle, the tumor volume of the HL085 + IN10018 + PDL1 antibody (1+25+10 mg/kg) group was always smaller than that of the PD-L1 antibody (10 mg/kg) group and the HL085 + IN10018 (1+25mg/kg) group, and on the last day, that is, the 21st day of group administration, there were statistical differences compared with the both groups. It had a better effect in inhibiting tumor growth than the PD-L1 antibody (10 mg/kg) monotherapy group and the HL085 + IN10018 (1+25 mg/kg) two-drug combination group.

### Example 6: Study on the induction of in vitro immunogenic cell death targets of mouse breast cancer 4T1 cells by the test compounds Cobimetinib and AMP-945

### Assay materials:

### 1) Drugs used in this assay

Cobimetinib was provided by MCE, Lot No.: 07394

AMP -945 was provided by MCE, Lot No.: 143253

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

4T1 cells (source: Nanjing Kebai Biotechnology Co., Ltd., catalog number: CBP60352) were cultured in RPMI 1640 (Shanghai Yuanpei, Cat No.: L210KJ, Lot No.: F210916) + 10% FBS (Gibco, Cat No.: 10099-141c, Lot No.: 2158737cp) at conditions of 37 °C, 5% CO₂. Trypsin was used two to three times a week for routine digestion and subculture. When the cells were in the exponential growth phase and adhered to the wall and confluent to 80%-90%, the cells were harvested and plated. 4T1 cells were digested with trypsin and then harvested and counted. According to the counting results, the cells were diluted with RPMI1640 + 10% FBS to a dilution concentration of 50,000 cells per ml, and then plated in 12-well cell culture plates, with 2 ml of cell suspension per well, i.e. 100,000 cells. After plating, the cells were cultured in a 37 °C, 5% CO₂ incubator. After 24 hours of cell spreading, six groups were set up. The first group was the control group, to which culture medium was added, the second group was AMP-945 at a concentration of 3 µM, the third group was AMP-945 at a concentration of 6 µM, the fourth group was Cobimetinib (MCE) at a concentration of 3uM, the fifth group was AMP-945 (3 µM) combined with Cobimetinib (3 µM), and the sixth group was AMP-945 (6 µM) combined with Cobimetinib (3 µM). The drugs were mixed and cultured in a 5% CO₂ incubator at 37°C for 48 hours.

### Assay results

After 48 hours of drug action, cells were collected for flow analysis, and washed twice with flow buffer (PBS + 2% FBS) and 0.5 µl AF647. Anti-calreticulin antibody (abcam) was added to each well and mixed. The cells were incubated at 4 °C in the dark, flow buffer was added after 20 min, the annexin staining kit (Beyotime) was used and 195 µl Annexin-V-FITC conjugate was added. After pipetting and mixing with the cells, 5 µl Annexin-V-FITC antibody was added and gently mixed. Finally, 10 µl PI dye was added and mixed. The mixture was incubated at room temperature in the dark for 15 min, and the samples were sent to the flow cytometer for signal measurement.

Flow cytometry analysis results showed that the CRT positive rate and Annexin-V positive rate of the two-drug combination group were significantly better than those of the monotherapy group and the control group, see Fig. 15a and Fig. 15b.

### Example 7: Study on the induction of in vitro immunogenic cell death target in mouse colorectal cancer CT 26 cells by the test compounds Cobimetinib and AMP-945

### Assay materials:

### 1) Drugs used in this assay

Cobimetinib was provided by MCE, Lot No.: 07394
AMP -945 was provided by MCE, Lot No.: 143253

### 2) Antibodies used in this assay

Recombinant Alexa Fluor^{®} 647 fluorescent Anti-Calreticulin antibody (Abcam, Cat No.: ab196159, Lot No.: CR33676773). Annexin V-apoptosis detection kit (Beyotime, Cat No.: C1062L, Lot No.: 122221220706).

### Assay method:

CT26 cells (source: Nanjing Kebai Biotechnology Co., Ltd., catalog number: CBP60043) were cultured in RPMI 1640 (Shanghai Yuanpei, Cat No.: L210KJ, Lot No.: F210916) + 10% FBS (Gibco, Cat No.: 10099-141c, Lot No.: 2158737cp) under conditions of 37 °C, 5% CO₂.Trypsin was used two to three times a week for routine digestion and subculture. When the cells were in the exponential growth phase and adhered to the wall and confluent to 80%-90%, the cells were harvested and plated. After CT26 cells were digested with trypsin, the cells were harvested and counted. According to the counting results, the cells were diluted with RPMI1640 + 10% FBS to a dilution concentration of 50,000 cells per ml, and then plated in 12-well cell culture plates, with 2 ml of cell suspension per well, i.e. 100,000 cells. After plating, the cells were cultured in a 37 °C, 5% CO₂ incubator. After 24 hours of cell spreading, six groups were set up. The first group was the control group, to which culture medium was added, the second group was AMP-945 at a concentration of 0.75 µM, the third group was AMP-945 at a concentration of 1.5 µM, the fourth group was Cobimetinib (MCE) at a concentration of 3 uM, the fifth group was AMP-945 (0.75 µM) combined with Cobimetinib (3 µM), and the sixth group was AMP-945 (1.5 µM) combined with Cobimetinib (3 µM). The drugs were mixed and cultured in a 5% CO₂ incubator at 37°C for 48 hours.

### Assay results

After 48 h of drug action, cells were collected for flow analysis and washed twice with flow buffer (PBS + 2% FBS) and 0.5 µl AF647. Anti-calreticulin antibody (Abcam) was added to each well and mixed. The cells were incubated at 4 °C in the dark, flow buffer was added after 20 min, the annexin staining kit (Beyotime) was used and 195 µl Annexin-V-FITC conjugate was added. After pipetting and mixing with the cells, 5 µl Annexin-V-FITC antibody was added and gently mixed. Finally, 10 µl PI dye was added and mixed. The mixture was incubated at room temperature in the dark for 15 min, and the samples were sent to the flow cytometer for signal measurement.

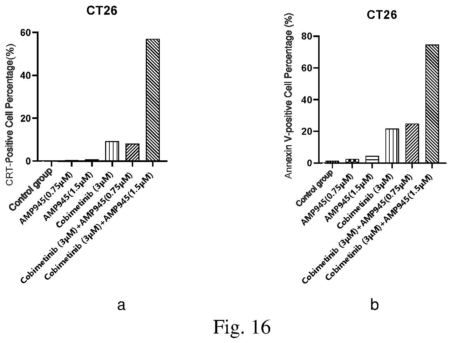

Flow cytometry analysis results showed that the CRT positive rate and Annexin-V positive rate of the two-drug combination group were significantly better than those of the monotherapy group and the control group, see Fig. 16a and Fig. 16b.

All references mentioned in this disclosure are incorporated by reference in their entirety as if each individual document were individually set forth. It should be understood that after reading the disclosure of the present disclosure, those skilled in the art can make various changes or modifications to the present disclosure, and these equivalent forms also fall within the scope defined by the appended claims of this application.

## Claims

1. Use of an FAK inhibitor, an MEK inhibitor and an immune checkpoint inhibitor in the manufacture of a medicament for treating tumors in a subject, wherein the FAK inhibitor, the MEK inhibitor and the immune checkpoint inhibitor are administered to the subject simultaneously or sequentially.

2. A pharmaceutical combination product of an FAK inhibitor, an MEK inhibitor and an immune checkpoint inhibitor for use in treating tumors in a subject, wherein the FAK inhibitor, the MEK inhibitor and the immune checkpoint inhibitor are administered to the subject simultaneously or sequentially.

3. A method for treating tumors, the method comprising administering a therapeutically effective amount of an FAK inhibitor, an MEK inhibitor and an immune checkpoint inhibitor to a subject in need thereof simultaneously or sequentially.

4. The use, pharmaceutical combination product or method according to any one of claims 1 to 3, wherein the FAK inhibitor is IN10018, Defactinib, GSK2256098, PF-00562271, VS-4718, APG-2449, AMP945, AMP886 or a pharmaceutically acceptable salt thereof, alternatively the FAK inhibitor is IN10018, Defactinib, AMP945 or a pharmaceutically acceptable salt thereof, further alternatively the FAK inhibitor is IN10018 or a pharmaceutically acceptable salt thereof, especially IN10018 tartrate, wherein the IN10018 has a structure of:

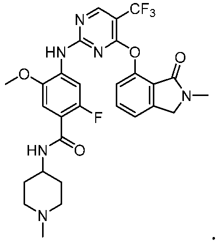

5. The use, pharmaceutical combination product or method according to any one of claims 1 to 4, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.

6. The use, pharmaceutical combination product or method according to any one of claims 1 to 5, wherein the immune checkpoint inhibitor is an anti-PD-1/PD-L1 antibody, a PD-1/PD-L1 small molecule inhibitor or a TIGIT inhibitor.

7. The use, pharmaceutical combination product or method according to any one of claims 1 to 6, wherein the FAK inhibitor is IN10018 or a pharmaceutically acceptable salt thereof, the MEK inhibitor is cobimetinib, HL-085, E-6201, Pimasertib, Refametinib, FCN-159 or a pharmaceutically acceptable salt thereof, and the immune checkpoint inhibitor is an anti-PD-1/PD-L1 antibody or a PD-1/PD-L1 small molecule inhibitor, especially atezolizumab.

8. The use, pharmaceutical combination or method according to any one of claims 1 to 7, wherein the tumor is Hodgkin's lymphoma, non-Hodgkin's lymphoma, non-small cell lung cancer, small cell lung cancer, hepatocellular carcinoma, bile duct cancer, myelodysplastic syndrome, acute lymphocytic leukemia, acute myeloid leukemia, chronic myeloid leukemia, thyroid cancer, glioma, colon cancer, rectal cancer, colorectal cancer, ovarian cancer, bladder cancer, prostate cancer, breast cancer, liposarcoma, fibrosarcoma, rhabdomyosarcoma, leiomyosarcoma, angiosarcoma, neuroblastoma, renal cell carcinoma, head and neck cancer, gastric cancer, esophageal cancer, cancer of the gastroesophageal junction, thymic cancer, pancreatic cancer, endometrial cancer, cervical cancer, melanoma, uveal melanoma, skin cancer, germ cell cancer, nasopharyngeal cancer, oropharyngeal cancer, or laryngeal cancer; further, the tumor is acute myeloid leukemia, melanoma, thyroid cancer, colon cancer, esophageal cancer, hepatocellular carcinoma, ovarian cancer, fibrosarcoma, gastric cancer, non-small cell lung cancer or bile duct cancer; yet further, the tumor is acute myeloid leukemia, melanoma, uveal melanoma, pancreatic cancer, ovarian cancer, colon cancer or colorectal cancer.

9. A kit or a pharmaceutically acceptable composition comprising:
(a) an FAK inhibitor;
(b) an MEK inhibitor; and
(c) an immune checkpoint inhibitor.

10. The kit or composition according to claim 9, wherein the FAK inhibitor is IN10018, Defactinib, GSK2256098, PF-00562271, VS-4718, APG-2449, AMP945, AMP886 or a pharmaceutically acceptable salt thereof, alternatively the FAK inhibitor is IN10018, Defactinib, AMP945 or a pharmaceutically acceptable salt thereof, further alternatively the FAK inhibitor is IN10018 or a pharmaceutically acceptable salt thereof, especially IN10018 tartrate, wherein the IN 10018 has a structure of:

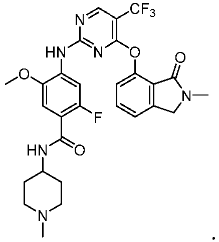

11. The kit or composition according to claim 9 or 10, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively, HL-085 or a pharmaceutically acceptable salt thereof.

12. The kit or composition according to any one of claims 9 to 11, wherein the immune checkpoint inhibitor is an anti-PD-1/PD-L1 antibody, a PD-1/PD-L1 small molecule inhibitor or a TIGIT inhibitor.

13. The kit or composition according to any one of claims 9 to 12, wherein the FAK inhibitor is IN10018 or a pharmaceutically acceptable salt thereof, the MEK inhibitor is cobimetinib, HL-085 or a pharmaceutically acceptable salt thereof, and the immune checkpoint inhibitor is an anti-PD-1/PD-L1 antibody or a PD-1/PD-L1 small molecule inhibitor, especially atezolizumab.

14. A kit or composition according to any one of claims 9 to 13 for use as a medicament.

15. Use of an FAK inhibitor and an MEK inhibitor in the manufacture of a medicament for treating tumors, wherein the FAK inhibitor is used to enhance the immunogenic cell death induced by the MEK inhibitor.

16. An FAK inhibitor for use in enhancing the immunogenic cell death induced by the MEK inhibitor in the treatment of tumors.

17. A method for treating tumors, comprising administering to a subject in need thereof a therapeutically effective amount of an FAK inhibitor and an MEK inhibitor simultaneously or sequentially, wherein the FAK inhibitor is used to enhance the immunogenic cell death induced by the MEK inhibitor.

18. The use, FAK inhibitor or method according to any one of claims 15 to 17, wherein the MEK inhibitor is trametinib, cobimetinib, bimetinib, Selumetinib, PD-325901, TAK-733, HL-085, E-6201, Pimasertib, Refametinib, FCN-159 or a pharmaceutically acceptable salt thereof, alternatively the MEK inhibitor is cobimetinib, HL-085 or a pharmaceutically acceptable salt thereof.

19. The use, FAK inhibitor or method according to any one of claims 15 to 18, wherein the FAK inhibitor is IN10018, Defactinib, GSK2256098, PF-00562271, VS-4718, APG-2449, AMP945, AMP886 or a pharmaceutically acceptable salt thereof, alternatively the FAK inhibitor is IN10018, Defactinib, AMP945 or a pharmaceutically acceptable salt thereof, further alternatively the FAK inhibitor is IN10018 or a pharmaceutically acceptable salt thereof, especially IN10018 tartrate, wherein the IN10018 has a structure of:
